(19) Europäisches Patentamt
European Patent Office
Office européen des brevets

(11) Publication number: **0 059 291**
**B1**

(12) **EUROPEAN PATENT SPECIFICATION**

(45) Date of publication of patent specification: **27.03.85**

(21) Application number: **81306136.3**

(22) Date of filing: **24.12.81**

(51) Int. Cl.$^4$: **C 07 D 471/04,** A 61 K 31/435 // (C07D471/04, 221:00, 221:00)

(54) **Antihypertensive agents.**

(30) Priority: **27.02.81 US 238911**
**31.08.81 US 297645**

(43) Date of publication of application:
**08.09.82 Bulletin 82/36**

(45) Publication of the grant of the patent:
**27.03.85 Bulletin 85/13**

(84) Designated Contracting States:
**AT BE CH DE FR IT LI LU NL SE**

(56) References cited:
**GB-A-1 022 214**
**GB-A-1 566 693**

(73) Proprietor: **AMERICAN HOME PRODUCTS CORPORATION**
**685, Third Avenue**
**New York, New York 10017 (US)**

(72) Inventor: **Sulkowski, Theodore Sylvester**
**316, Rockland Road**
**Wayne Pennsylvania (US)**
Inventor: **Bergey, James Laverne**
**117 Shady Lane**
**Lansdale Pennsylvania (US)**
Inventor: **Mascitti, Albert Angelo**
**1602 Daws Road**
**Norristown Pennsylvania (US)**

(74) Representative: **Porter, Graham Ronald et al**
**C/O John Wyeth & Brother Limited**
**Huntercombe Lane South Taplow**
**Maidenhead Berkshire, SL6 0PH (GB)**

EP 0 059 291 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Pharmacological agents possessing the ability to block cellular transmembrane influx of calcium are capable of suppressing that portion of myocardial or smooth muscle contractility which is dependent upon extracellular calcium. These pharmacological agents, termed calcium antagonists, have been proven to be useful in the treatment of hypertension, cardiac arrhythmias, angina pectoris, cardiac myopathy and coronary artery vasospasm (a possible cause of sudden cardiac death syndrome). Can J. Physiol, Pharmacol., *57* 443 (1979); Drugs, *15* 169 (1978); Acta Pharmacol, Toxical., *43*, suppl. 1, 45 (1978).

In theory, calcium antagonists are thought to act by blocking calcium influx through discrete calcium channels (slow channels) in cell membranes. Various tissues exhibit relative differences in sensistivity toward the calcium blocking effect achieved by certain calcium antagonists, theoretically as a result of tissue specific differences in the calcium channels. Acta Pharmacol. Toxicol., *43*, 5, (1978); loc. cit. 291 (1978); Microvascular Res., *5*, 73 (1973); Am Rev. Pharmacol. Toxicol., *17* 149 (1977). Calcium channels of tissue which are most sensitive to calcium antagonist blockade are those which allow calcium influx only when the cell membranes are electrically depolarised. α-adrenergic receptor-activated calcium channels are relatively unaffected by these agents. Circ. Res., *46* 426 (1980). This observation provides basis for evaluation of calcium antagonism.

Thus, vascular smooth muscle tissue from the rabbit aorta can be made to contract when exposed to a depolarising solution containing an elevated potassium ion concentration and normal amounts of calcium ions. Calcium antagonists added to the solution produce a dose dependent relaxation of the contracted rabbit aortic tissue. Normal contraction of the aortic tissue can then be induced in the presence of a calcium antagonist by subsequent addition of an α-adrenergic agonist, such as norepinephrine, to the solution. Eur. J. Pharmacol., *53*, 281 (1979); Circ. Res., *46* 426 (1980); J. Exp. Pharmacol. Therap., *174* 500 (1970). The normal contraction produced by an α-adrenergic agonist after maximal smooth muscle relaxation has been induced by a calcium antagonist, serves to distinguish the calcium blocking effect of an agent from a nonspecific depressant effect on the muscle.

In accordance with this invention there is provided a group of new 1,4,5,6,7,8-hexahydro-2-alkyl-4-aryl-5-oxo-1,7-naphthyridine-3-carboxylic acid esters and pharmaceutically acceptable salts thereof, which compounds are calcium antagonists useful in the treatment of hypertension, cardiac arrhythmias, angina pectoris, cardiac myopathy and coronary artery vasospasm.

More specifically, the compounds of this invention are those of the formula:

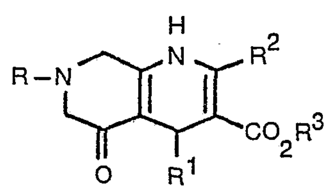

(I)

in which

R is hydrogen, alkyl of 1 to 6 carbon atoms or benzyl;

$R^2$ is alkyl of 1 to 6 carbon atoms;

$R^3$ is alkyl of 1 to 6 carbon atoms, alkoxyalkyl in which each alkyl moiety has 1 to 6 carbon atoms, $—CH_2CF_3$,

$$—CH_2CH_2CF_3 \text{ or } —CH_2(CH_2)_n—N\begin{matrix} / R^9 \\ \backslash R^{10} \end{matrix} \qquad (II)$$

where

$R^9$ is hydrogen or alkyl of 1 to 6 carbon atoms; and

$R^{10}$ is alkyl of 1 to 6 carbon atoms or arylalkyl of 7 to 10 carbon atoms or $R^9$ and $R^{10}$ taken with the nitrogen atom to which they are attached form a pyrrolidinyl, imidazolidinyl, piperidyl, piperazinyl, 4-alkylpiperazinyl in which the alkyl group contains from 1 to 6 carbon atoms or morpholinyl heterocycle; and

n is one of the integers 0, 1 or 2;

$R^1$ is pentafluorophenyl, tetrafluorophenyl or

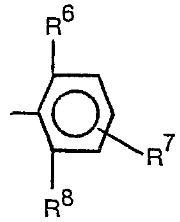

(III)

wherein

$R^6$ and $R^8$ are, independently, hydrogen, alkyl of 1 to 6 carbon atoms, halo, trifluoromethyl, alkoxy of 1 to 6 carbon atoms, alkylthio of 1 to 6 carbon atoms, cyano or nitro;

$R^7$ is hydrogen, alkyl of 1 to 6 carbon atoms, halo, trifluoromethyl, cyano or nitro; or $R^8$ is as defined above whilst $R^6$ and $R^7$ are in the ortho position to each other and taken together are butadienylene, tetramethylene or trimethylene; or a pharmaceutically acceptable salt thereof.

The compounds that are preferred because of production economics and availability of starting materials are those in which the aliphatic moieties are straight or branched chain containing from 1 to 4 carbon atoms, the arylalkyl group representing $R^{10}$ is benzyl or phenethyl and n is 1 or 2.

The compounds of this invention may be prepared by reacting a 3-oxo-5-hydroxy-1,2,3,6-tetrahydropyridine derivative (piperidine-3,5-dione), an aldehyde and a substituted or unsubstituted 3-aminocrotonate ester according to the reaction scheme

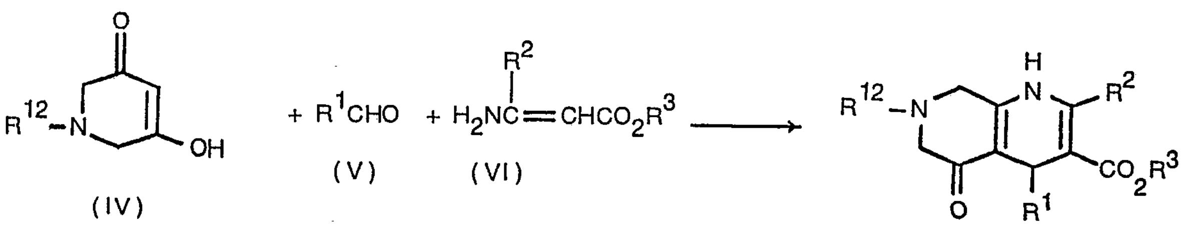

(IV) (V) (VI) (VII)

where $R^{12}$ is alkyl of 1 to 6 carbon atoms or benzyl. The terms "unsubstituted" and "substituted" as used herein in respect of the 3-aminocrotonate esters denote that $R^2$ is alkyl of 1 carbon atom and alkyl of 2 to 6 carbon atoms respectively. The benzyl group of the compound of formula VII or a salt thereof may be removed by hydrogenolysis to afford the 7-unsubstituted products.

If desired, the substituted or unsubstituted 3-aminocrotonate esters may be prepared, *in situ*, by ammonolysis of the alkanoylacetic acid having the formula $R^2COCH_2CO_2R^3$.

The compounds of this invention may also be prepared by reaction of a benzylidine alkanoylacetic acid ester of the formula:

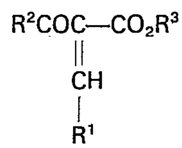

(VIII)

with a 3-oxo-5-hydroxy-1,2,3,6-tetrahydropyridine of the formula:

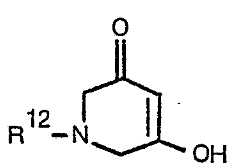

(IV)

in the presence of ammonia. The ammonia may be supplied to the reaction medium as gaseous ammonia, ammonium hydroxide or an ammonium salt of a weak organic acid such as ammonium acetate. The benzylidene alkanoylacetic acid ester reactant may be prepared by reaction of an aldehyde having the formula $R^1CHO$ with an alkanoylacetic acid ester of the formula $R^2COCH_2CO_2R^3$.

The 3-oxo-5-hydroxy-1,2,3,6-tetrahydropyridines having formula IV may be prepared from N-substituted glycine esters by standard procedures. The N-methyl and N-benzyl

3-oxo-5-hydroxy-1,2,3,6-tetrahydropyridines are literature compounds: Archiv. der Pharmazie, *300* 91 (1967); J.A.C.S. *95* 7458 (1973); Tet. Lett., *4075* (1977). The preparatory technique generally follows the equations:

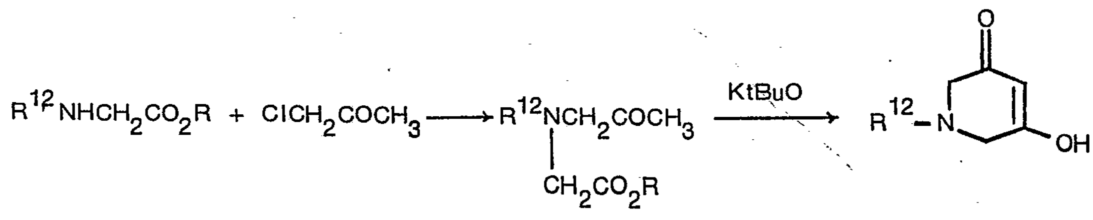


(IX) (X) (XI) (IV)

The aldehyde and substituted or unsubstituted 3-aminocrotonate reactants are either commercially available or may be prepared by standard procedures.

The pharmaceutically acceptable salts of the antihypertensive agents of this invention may be prepared directly by neutralisation of the free base or by metathetical displacement. The physiologically acceptable salts may be formed with organic or inorganic acids such as hydrochloric, hydrobromic, phosphoric, sulphuric, sulphonic, nitric, methylsulphonic, acetic, maleic, succinic, fumaric, tartaric, citric, salicylic, lactic, naphthalenedisulphonic acid, and the like. The free bases may be prepared by neutralizing the acid addition salts by treatment with a base.

The N-benzyl 1,4,5,6,7,8-hexahydro-2-alkyl-4-aryl-5-oxo-1,7-naphthyridine-3-carboxylic acid esters form an additional intermediate compound aspect of the invention useful in the preparation of the N-unsubstituted analogues referred to *supra*. The N-benzyl intermediates are depicted by the structural formula:

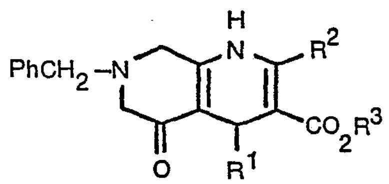

(XII)

where $PhCH_2$ is benzyl and the $R^1$—$R^3$ groups are defined *supra*. These compounds, although categorised as intermediates herein, are very potent *in vitro* $Ca^{+2}$ antagonists. The N-benzyl derivatives generally lack sufficient bioavailability *in vivo* (because of their insolubility, protein binding propensity or analogous functional deactivating property) to qualify as meaningful antihypertensive agents. Derivitisation of the 7-benzyl intermediates to afford *in vivo* antihypertensive activity is achieved by preparing compounds that are functionalised to provide solubility and avoid physical deactivation in the animal. Thus, preparation of the aminoalkyl esters (where $R^3$ has formula II) illustrates one method for improving the bioavailability of the 7-benzyl derivatives such that they afford excellent antihypertensive agents without removal of the 7-benzyl substituent. The *in vitro* $Ca^{+2}$ antagonist level and the *in vivo* blood pressure reduction obtained, where applicable, for these intermediates, is presented in the following examples.

The compounds of this invention were initially shown to exhibit $Ca^{+2}$ antagonism in rabbit aortic strips wherein the strips were contracted in an organ bath containing a modified physiological salt solution (Broekaert et al., Europ. J. Pharmacol. *53* 281 (1979)) in which 100 millimoles potassium ion had been substituted on an equimolar basis for sodium ion. After a stable active tension has developed in the strip, as measured by Statham UC-2 force transducers and recorded on an eight channel Beckman Dynograph Polygraphic Recorder, an amount of the antagonist was added to the organ bath to make a $10^{-5}$ molar concentration of antagonist. The depressant effect, expressed as percent relaxation, was taken from the mean of at least two experiments. After maximum $Ca^{+2}$ antagonist induced relaxation was obtained, a maximal dose of norepinephrine ($10^{-5}$ moles) was added to the organ bath to determine whether normal α-adrenergic responses were still effected and show that the compound being tested was not a general depressant. A $Ca^{+2}$ antagonist producing a 20 per cent relaxation of aortic tissue in this test procedure at a $10^{-5}$ molar bath concentration of the antagonist, generally produces a significant reduction blood pressure when a sufficient amount is given to the spontaneously hypertensive rat.

The *in vivo* blood pressure lowering ability of the compounds of this invention was established by measuring the systolic pressure of spontaneously hypertensive rats with a Decker Caudal Plethysmograph or similar sensor device. The compound being tested is administered to groups of four rats and their blood pressure is read prior to compound administration and at 1.5 and 4 hours after compound administration. Depending upon the behaviour of the compound being tested, the schedule of blood pressure readings and route of administration is modified. Initially the compounds are administered orally but where compound

solubility is a factor, the compounds may be administered parenterally (i.e. i.p., i.m., s.c., i.v., etc.) as desired. The compounds of this invention were initially administered orally at a standard testing dose of 50 mg/kg.

Based upon the activity profile elicited by the compounds of this invention in the above-described standard scientifically recognised test models, the compounds are established as hypotensive agents useful in the treatment of hypertension and conditions characterised by constrictive blood flow in coronary arteries. For that purpose, the compounds may be administered orally or parenterally in suitable dosage forms compatable with the route of administration, whether oral, intraperitoneal, intramuscular, intravenous, intranasal, buccal, etc. The effective dose range determined in the animal test models has been established at from 1 to about 50 milligrams per kilogram host body weight to be administered in single or plural doses as needed to obtain the desired hypotensive response. The specific dosage regimen for a given patient will depend upon age, pathological state, severity of dysfunction, size of the patient, etc. Oral administration is performed with either a liquid or solid dosage unit in any conventional form such as tablets, capsules, solutions, etc., which comprise a unit dose (e.g. from about 25 milligrams to about 4 grams) of the active ingredient alone or in combination with adjuvants needed for conventional coating, tableting, solubilising, flavour or colouring. Parenteral administration with liquid unit dosage forms may be via sterile solutions or suspensions in aqueous or oleagenous medium. Isotonic aqueous vehicle for injection is preferred with or without stabilisers, preservatives and emulsifiers.

Thus the invention also provides a pharmaceutical composition comprising a compound having formula I or a pharmaceutically acceptable salt thereof in association with a pharmaceutically acceptable carrier. The composition may be prepared by bringing the compound having formula I or a pharmaceutically acceptable salt thereof into association with the said carrier, for example, by mixing the ingredients.

The following examples illustrate the preparation of a representative number of compounds of this invention. After each example, the $Ca^{+2}$ antagonist activity of the compound is presented in terms of per cent relaxation (P.R.) at the stated concentration. Similarly, the anti-hypertensive activity is reported in terms of millimetres mercury (mmHg) blood pressure (B.P.) reduction at the stated time post 50 mg/kg oral dosing unless otherwise indicated.

Example 1

1,4,5,6,7,8-Hexahydro-2,7-dimethyl-4-(2-nitrophenyl)-5-oxo-1,7-naphthyridine-3-carboxylic acid methyl ester

1-Methyl-3-oxo-5-hydroxy-1,2,3,6-tetrahydropyridine (6.36 g.), 5.93 g. of methyl 3-aminocrotonate (97%), 7.56 g. of *o*-nitrobenzaldehyde and 100 ml. of ethanol were heated at reflux for 1.4 hours. The mixture was cooled to room temperature and the precipitated solid was separated by filtration. The solid, m.p. 233—235°C., was suspended in ethanol and saturated with hydrogen chloride. The solid dissolved, then reprecipitated. The mixture was cooled and the solid was separated by filtration. The solid was recrystallised from 95% ethanol with charcoal treatment to obtain the title compound as the hydrochloride, m.p. 227—229°C. dec.

Analysis for: $C_{18}H_{19}N_3O_5$ . HCl

| | |
|---|---|
| Calculated: | C, 54.90; H, 5.12; N, 10.67; Cl, 9.00 |
| Found: | C, 54.87; H, 5.20; N, 10.74; Cl, 8.96 |

P.R.=14 at $10^{-5}$ M

B.P.=−27 at 1.5 hours; −21 at 4 hours.

Example 2

1,4,5,6,7,8-Hexahydro-2,7-dimethyl-4-(2-methylphenyl)-5-oxo-1,7-naphthyridine-3-carboxylic acid methyl ester

A mixture of 7.65 g. of 1-methyl-3-oxo-5-hydroxy-1,2,3,6-tetrahydropyridine, 7.2 g. of *o*-tolualdehyde, 6.9 g. of methyl 3-aminocrotonate (97%), 110 ml. of ethanol and 35 ml. of acetic acid was refluxed for 3 hours. The mixture was evaporated to dryness *in vacuo*. The residue was dissolved in dichloromethane and extracted with saturated sodium carbonate solution. The dichloromethane solution was evaporated to dryness. The residue was slurried with diethyl ether and the solid was separated by filtration. Recrystallisation from ethanol afforded the title compound, m.p. 249—252°C. dec.

Analysis for: $C_{19}H_{22}N_2O_3$

| | |
|---|---|
| Calculated: | C, 69.91; H, 6.80; N, 8.58 |
| Found: | C, 70.07; H, 6.81; N, 8.73 |

The solid was suspended in ethanol and saturated with hydrogen chloride. The solvent was removed *in vacuo*. The residue was recrystallised twice from ethanol-diethyl ether to obtain the hydrochloride, m.p. 245—247°C. dec.

Analysis for: $C_{19}H_{22}N_2O_3$ . HCl

| | |
|---|---|
| Calculated: | C, 62.89; H, 6.39; N, 7.72; Cl, 9.77 |
| Found: | C, 62.48; H, 6.36; N, 7.72; Cl 9.75 |

P.R.=31 at $10^{-5}$ M
B.P.=−57 at 1.5 hours; −28 at 4 hours.

Example 3
1,4,5,6,7,8-Hexahydro-2-methyl-4-(2-methylphenyl)-5-oxo-7-(phenylmethyl)-1,7-naphthyridine-3-carboxylic acid methyl ester

A mixture of 6.1 g. of 1-benzyl-3-oxo-5-hydroxy-1,2,3,6-tetrahydropyridine, 3.6 g. of methyl 3-aminocrotonate (97%), 3.6 g of *o*-tolualdehyde, 55 ml. of ethanol and 18 ml. of acetic acid was refluxed for 7 hours. The solution was evaporated to dryness *in vacuo*. The residue was triturated with diethyl ether and the solid was separated by filtration. Recrystallisation from ethanol afforded the title compound, m.p. 201—204°C.

Analysis for: $C_{25}H_{26}N_2O_3$

| | |
|---|---|
| Calculated: | C, 74.60; H, 6.51; N, 6.96 |
| Found: | C, 74.42; H, 6.57; N, 6.87 |

P.R.=90 at $10^{-6}$ M
B.P.=−23 at 1.5 hours; −41 at 4 hours.

Example 4
1,4,5,6,7,8-Hexahydro-2,7-dimethyl-5-oxo-4-[2-(trifluoromethyl)phenyl]-1,7-naphthyridine-3-carboxylic acid methyl ester

1-Methyl-3-oxo-5-hydroxy-1,2,3,6-tetrahydropyridine (6.36 g.) 5.93 g. of methyl 3-aminocrotonate, 8.70 g. of *o*-trifluoromethylbenzaldehyde and 100 ml. of ethanol were refluxed for 2 hours. The reaction mixture was filtered and allowed to cool to room temperature. The solid was separated by filtration. The solid, m.p. 250—252°C. dec., was suspended in ethanol and saturated with hydrogen chloride. The solution was evaporated to dryness *in vacuo*. The residue was slurried with diethyl ether and filtered. Recrystallisation from ethanol-di-ethyl ether afforded the title compound as the hydrochloride, m.p. 234—237°C. dec.

Analysis for: $C_{19}H_{19}N_2F_3O_3$ . HCl

| | |
|---|---|
| Calculated: | C, 54.74; H, 4.84; N, 6.72; Cl, 8.50 |
| Found: | C, 54.42; H, 4.90; N, 6.64; Cl, 8.46 |

P.R.=48 at $10^{-5}$ M
B.P.=−68 at 1.5 hours; −70 at 4 hours.

Example 5
1,4,5,6,7,8-Hexahydro-2-methyl-5-oxo-7-(phenylmethyl)-4-[2-(trifluoromethyl)phenyl]-1,7-naphthyridine-3-carboxylic acid methyl ester

A mixture of 8.5 g. of 1-benzyl-3-oxo-5-hydroxy-1,2,3,6-tetrahydropyridine, 4.75 g. of methyl 3-aminocrotonate (97%), 6.96 g. of *o*-trifluoromethylbenzaldehyde and 125 ml. of ethanol was refluxed for 2 hours. The mixture was filtered while hot and the filtrate was allowed to cool to room temperature. The solid was separated by filtration. Recrystallisation from ethanol afforded the title compound, m.p. 225—227°C.

Analysis for: $C_{25}H_{23}N_2F_3O_3$

| | |
|---|---|
| Calculated: | C, 65.78; H, 5.08; N, 6.14 |
| Found: | C, 65.68; H, 5.18; N, 6.04 |

P.R.=40 at $10^{-6}$ M

Example 6
1,4,5,6,7,8-Hexahydro-2,7-dimethyl-4-(3-nitrophenyl)-5-oxo-1,7-naphthyridine-3-carboxylic acid methyl ester

1-Methyl-3-oxo-5-hydroxy-1,2,3,6-tetrahydropyridine (5.10 g.), 4.75 g. of methyl 3-aminocrotonate (97%), 6.05 g. of *m*-nitrobenzaldehyde and 100 ml. of ethanol were refluxed for 1.5 hours. The mixture was filtered while hot and allowed to cool to room temperature. The solid was separated by filtration. The solid, m.p. 210°C. dec., was suspended in ethanol and saturated with hydrogen chloride. The solution was filtered and evaporated to dryness. The residue was slurried with diethyl ether and filtered. Two recrystallisations from ethanol afforded the title compound as the hydrochloride, m.p. 220°C. dec.

Analysis for: $C_{18}H_{19}N_3O_5$ . HCl

| | |
|---|---|
| Calculated: | C, 54.90; H, 5.12; N, 10.67; Cl, 9.00 |
| Found: | C, 54.89; H, 5.19; N, 10.76; Cl, 8.95 |

P.R.=35 at $10^{-5}$ M
B.P.= −85 at 1.5 hours; −47 at 4 hours.

Example 7

1,4,5,6,7,8-Hexahydro-2-methyl-4-(2-methylphenyl)-5-oxo-1,7-naphthyridine-3-carboxylic acid methyl ester

Twelve grams of 1,4,5,6,7,8-hexahydro-2-methyl-4-(2-methylphenyl)-5-oxo-7-(phenylmethyl)-1,7-naphthyridine-3-carboxylic acid methyl ester (Example 3) were suspended in 150 ml. of 95% ethanol and 2.5 ml of concentrated hydrochloric acid. One half gram of 10% palladium on carbon was added and the mixture was shaken with hydrogen at an initial pressure of 3.45 bars (50 psi). After 7.5 hours, the mixture was filtered to remove the catalyst. The filtrate was evaporated to dryness *in vacuo*. The residue was slurried with ethanol and filtered. Recrystallisation from 95% ethanol afforded the title compound as the hydrochloride, m.p. 278—280°C. dec.

Analysis for: $C_{18}H_{20}N_2O_3$ . HCl

| | |
|---|---|
| Calculated: | C, 61.97; H, 6.07; N, 8.03; Cl, 10.18 |
| Found: | C, 62.02; H, 6.19; N, 8.23; Cl, 9.96 |

P.R.=46 at $10^{-5}$ M

B.P.= −77 at 1.5 hours; −64 at 4 hours.

Example 8

4-(2-Chlorophenyl)-1,4,5,6,7,8-hexahydro-2,7-dimethyl-5-oxo-1,7-naphthyridine-3-carboxylic acid methyl ester

1-Methyl-3-oxo-5-hydroxy-1,2,3,6-tetrahydropyridine (5.10 g.), 4.75 g. of methyl 3-aminocrotonate, 5.62 g. of *o*-chlorobenzaldehyde and 85 ml. of ethanol were heated to reflux. Solid began to precipitate after 15 minutes. Refluxing was continued for 45 minutes. The mixture was cooled and the solid was separated by filtration. The solid, m.p. 253—255°C. dec., was suspended in ethanol and saturated with hydrogen chloride.

The solution was evaporated *in vacuo*. The residue was slurried with diethyl ether and filtered. Two recrystallisations from ethanol-diethyl ether afforded the title compound as the hydrochloride, m.p. 241—243°C. dec.

Analysis for: $C_{18}H_{19}N_2ClO_3$ . HCl

| | |
|---|---|
| Calculated: | C, 56.40; H, 5.26; N, 7.31; Cl, 18.46 |
| Found: | C, 56.21; H, 5.30; N, 7.19; Cl, 18.17 |

P.R.=28.3 at $10^{-5}$ M

B.P.= −54 at 1.5 hours; −30 at 4 hours.

Example 9

4-(2,6-Dichlorophenyl)-1,4,5,6,7,8-hexahydro-2,7-dimethyl-5-oxo-1,7-naphthyridine-3-carboxylic acid methyl ester

A mixture of 7.25 g. of 1-methyl-3-oxo-5-hydroxy-1,2,3,6-tetrahydropyridine, 8.75 g. of 2,6-dichlorobenzaldehyde, 5.94 g. of methyl 3-aminocrotonate (97%) and 135 ml. of ethanol were heated at reflux for 3 hours. The mixture was filtered and the filtrate was evaporated to dryness. The residue was slurried with ethanol and the solid was separated by filtration. The solid, m.p. 258—260°C. dec., was suspended in ethanol and saturated with hydrogen chloride. The solution was evaporated to dryness. The residue was triturated with a small volume of ethanol and filtered. The solid was recrystallised from 95% ethanol to obtain the title compound as the hydrochloride, m.p. 260—262°C. dec.

Analysis for: $C_{18}H_{18}Cl_2N_2O_3$ . HCl

| | |
|---|---|
| Calculated: | C, 51.75; H, 4.58; N, 6.71; Cl, 25.66 |
| Found: | C, 51.71; H, 4.67; N, 7.06; Cl, 24.77 |

P.R.=41.5 at $10^{-5}$ M

B.P.= −22 at 1.5 hours; −43 at 4 hours.

Example 10

1,4,5,6,7,8-Hexahydro-2-methyl-4-(3-nitrophenyl)-5-oxo-7-(phenylmethyl)-1,7-naphthyridine-3-carboxylic acid methyl ester

1-Benzyl-3-oxo-5-hydroxy-1,2,3,6-tetrahydropyridine (10.6 g.), 5.45 g. of methyl 3-aminocrotonate, 7.55 g. of *m*-nitrobenzaldehyde and 120 ml. of ethanol were heated at reflux for 3 hours. The mixture was filtered and the filtrate was cooled in an ice bath. The precipitated solid was separated by filtration. The solid, m.p. 189—191°C dec., was suspended in ethanol and saturated with hydrogen chloride. The salt was separated and recrystallised from aqueous ethanol to obtain the title compound as the hydrochloride, m.p. 211—214°C dec.

Analysis for: $C_{24}H_{23}N_3O_5$ . HCl

| | |
|---|---|
| Calculated: | C, 61.34; H, 5.15; N, 8.94; Cl, 7.54 |
| Found: | C, 60.99; H, 5.32; N, 8.71; Cl, 7.56 |

P.R.=89.7 at $10^{-6}$ M

Example 11

4-(2,5-Dimethylphenyl)-1,4,5,6,7,8-hexahydro-2,7-dimethyl-5-oxo-1,7-naphthyridine-3-carboxylic acid methyl ester

A mixture of 5.10 g. of 1-methyl-3-oxo-5-hydroxy-1,2,3,6-tetrahydropyridine, 4.75 g. of methyl 3-aminocrotonate, 5.38 g. of 2,5-dimethylbenzaldehyde and 100 ml. of ethanol was refluxed for 1.5 hours. The mixture was filtered and allowed to cool to room temperature. The solid was separated by filtration. The solid, m.p. 270°C dec, was suspended in ethanol and saturated with hydrogen chloride. The solution was evaporated to dryness *in vacuo.* The residue was recrystallised from 95% ethanol to obtain the title compound as the hydrochloride, m.p. 243—246°C dec.

Analysis for: $C_{20}H_{24}N_2O_3$ . HCl

| | |
|---|---|
| Calculated: | C, 63.73; H, 6.68; N, 7.43; Cl, 9.41 |
| Found: | C, 63.52; H, 6.60; N, 7.46; Cl, 9.39 |

P.R.=22 at $10^{-5}$ M

Example 12

1,4,5,6,7,8-Hexahydro-2-methyl-5-oxo-4-[2-(trifluoromethyl)phenyl]-1,7-naphthyridine-3-carboxylic acid methyl ester

The hydrochloride prepared from 11.5 g. of 1,4,5,6,7,8-hexahydro-2-methyl-5-oxo-7-(phenylmethyl)-4-[2-(trifluoromethyl)phenyl]-1,7-naphthyridine-3-carboxylic acid methyl ester (Example 5) was dissolved in 150 ml. of 90% methanol. One gram of 10% palladium on carbon was added and the mixture was shaken with hydrogen at an initial pressure of 3.45 bars (50 psi). After 3 hours, the mixture was filtered to remove the catalyst. The filtrate was evaporated to dryness. The residue was slurried with diethyl ether and filtered. Recrystallisation from methanol-diethyl ether afforded the title compound as the hydrochloride, m.p. 272—275°C dec.

Analysis for: $C_{18}H_{17}N_2F_3O_3$ . HCl

| | |
|---|---|
| Calculated: | C, 53.67; H, 4.50; N, 6.95; Cl, 8.80 |
| Found: | C, 53.64; H, 4.54; N, 6.85; Cl, 8.74 |

P.R.=89.1 at $10^{-5}$ M

B.P.= −88 at 1.5 hours

Example 13

1,4,5,6,7,8-Hexahydro-2-methyl-4-(3-nitrophenyl)-5-oxo-1,7-naphthyridine-3-carboxylic acid methyl ester

1,4,5,6,7,8-Hexahydro-2-methyl-4-(3-nitrophenyl)-5-oxo-7-(phenylmethyl)-1,7-naphthyridine-3-carboxylic acid methyl ester hydrochloride (8.5 g.) (Example 10) was dissolved in 250 ml. of 90% methanol. One-half gram of 10% palladium on carbon was added and the mixture was shaken with hydrogen at atmospheric pressure. Hydrogen uptake ceased after 45 minutes. The mixture was filtered to remove the catalyst and the filtrate was concentrated to dryness *in vacuo.* The residue was crystallised from isopropanol. Recrystallisation from ethanol afforded the title compound as the hydrochloride, hemihydrate, m.p. 244°C dec.

Analysis for: $C_{17}H_{17}N_3O_5$ . HCl. $\frac{1}{2}$ $H_2O$

| | |
|---|---|
| Calculated: | C, 52.52; H, 4.92; N, 10.81; Cl, 9.12 |
| Found: | C, 52.46; H, 4.82; N, 11.06; Cl, 9.68 |

P.R.=86.5 at $10^{-5}$ M

B.P.=(Dose 25 mg/kg) −58 at 1.5 hours.

Example 14

1,4,5,6,7,8-Hexahydro-2-methyl-5-oxo-4-[3-(trifluoromethyl)phenyl]-1,7-naphthyridine-3-carboxylic acid methyl ester

A mixture of 10.5 g. of 1-benzyl-3-oxo-5-hydroxy-1,2,3,6-tetrahydropyridine, 5.95 g. of methyl 3-aminocrotonate (97%), 8.7 g. of 3-trifluoromethylbenzaldehyde and 120 ml. of ethanol was refluxed for 3 hours. The solution was concentrated to dryness. The residue was triturated with ethanol and the solid was separated by filtration. The solid was suspended in methanol and saturated with hydrogen chloride. The solid was separated and recrystallised from ethanol to obtain 1,4,5,6,7,8-hexahydro-2-methyl-5-oxo-7-(phenylmethyl)-4-[3-(trifluoromethyl)phenyl]-1,7-naphthyridine-3-carboxylic acid methyl ester hydrochloride, m.p. 225°C dec.

Analysis for: $C_{25}H_{23}N_2F_3O_3$ . HCl

| | |
|---|---|
| Calculated: | C, 60.92; H, 4.91; N, 5.68; Cl, 7.19 |
| Found: | C, 60.76; H, 5.07; N, 5.58; Cl, 7.52 |

P.R.=46.2 at $10^{-6}$ M

Nine grams of the above hydrochloride, 0.5 g. of 10% palladium on carbon and 250 ml. of methanol were shaken with hydrogen at an initial pressure of 2.76 bars (40 psi). After 2 hours, the mixture was filtered

to remove the catalyst. The filtrate was evaporated to dryness *in vacuo* and the residue crystallised on standing overnight. Recrystallisation from ethyl acetate—diethyl ether afforded the title compound as the hydrochloride, m.p. 232—234°C dec.

Analysis for: $C_{18}H_{17}N_2F_3O_3 . HCl$

| | |
|---|---|
| Calculated: | C, 53.67; H, 4.50; N, 6.95; Cl, 8.80 |
| Found: | C, 53.65; H, 4.67; N, 6.99; Cl, 8.83 |

P.R.=84.0 at $10^{-5}$ M
P.B.= −51 at 1.5 hours; −43 at 4 hours.

Example 15

4-(2-Fluorophenyl)-1,4,5,6,7,8-hexahydro-2-methyl-5-oxo-1,7-naphthyridine-3-carboxylic acid methyl ester

A mixture of 10.5 g. of 1-benzyl-3-oxo-5-hydroxy-1,2,3,6-tetrahydropyridine, 5.95 g. of methyl 3-aminocrotonate (97%), 6.2 g. of 2-fluorobenzaldehyde and 125 ml. of ethanol was refluxed for 3 hours. The precipitated solid was separated by filtration. The solid was suspended in methanol and saturated with hydrogen chloride. The solid was separated and recrystallised from 95% ethanol to obtain 4-(2-fluorophenyl)-1,4,5,6,7,8-hexahydro-2-methyl-5-oxo-7-(phenylmethyl)-1,7-naphthyridine-3-carboxylic acid methyl ester hydrochloride, m.p. 237°C dec.

Analysis for: $C_{24}H_{23}N_2FO_3 . HCl$

| | |
|---|---|
| Calculated: | C, 65.08; H, 5.46; N, 6.32; Cl, 8.00 |
| Found: | C, 64.97; H, 5.57; N, 6.31; Cl, 8.05 |

P.R.=48 at $10^{-6}$ M.

Ten grams of the above hydrochloride, 0.5 g. of 10% palladium on carbon, 200 ml. of methanol and 15 ml. of water were shaken with hydrogen at an initial pressure of 2.76 bars (40 psi). After 2 hours, the catalyst was separated by filtration and the filtrate was evaporated to dryness *in vacuo*. The solid residue was slurried with acetonitrile, filtered, and dried *in vacuo*. Recrystallisation from 95% ethanol afforded the title compound as the hydrochloride, m.p. 262—263°C dec.

Analysis for: $C_{17}H_{17}N_2FO_3 . HCl$

| | |
|---|---|
| Calculated: | C, 57.88; H, 5.14; N, 7.94; Cl, 10.05 |
| Found: | C, 57.58; H, 5.15; N, 7.41; Cl, 10.05 |

P.R.=54 at $10^{-5}$ M
B.P.= −45 at 1.5 hours.

Example 16

4-(3-Cyanophenyl)-1,4,5,6,7,8-hexahydro-2,7-dimethyl-5-oxo-1,7-naphthyridine-3-carboxylic acid methyl ester

1-Methyl-3-oxo-5-hydroxy-1,2,3,6-tetrahydropyridine (6.6 g.), 5.95 g. of methyl 3-aminocrotonate (97%), 6.5 g. of 3-cyanobenzaldehyde and 125 ml. of ethanol were refluxed for 3 hours. The solvent was removed *in vacuo* and the residue was crystallised from a small volume of ethanol. The solid was suspended in methanol and saturated with hydrogen chloride. The solution was evaporated to dryness. The residue crystallised on standing in a small volume of ethanol. The solid was recrystallised from ethanol to obtain the title compound as the hydrochloride, m.p. 226—229°C dec.

Analysis for: $C_{19}H_{19}N_3O_3 . HCl$

| | |
|---|---|
| Calculated: | C, 61.05; H, 5.39; N, 11.24; Cl, 9.48 |
| Found: | C, 60.09; H, 5.66; N, 11.16; Cl, 9.36 |

Example 17

4-(2,3-Dimethylphenyl)-1,4,5,6,7,8-hexahydro-2,7-dimethyl-5-oxo-1,7-naphthyridine-3-carboxylic acid methyl ester

1-Methyl-3-oxo-5-hydroxy-1,2,3,6-tetrahydropyridine (6.36 g.), 5.93 g. of methyl 3-aminocrotonate (97%), 11 g. of 2,3-dimethylbenzaldehyde and 100 ml. of ethanol were refluxed for 3 hours. The mixture was filtered while hot and then left standing overnight. The precipitated solid was separated by filtration. The solid was suspended in methanol and saturated with hydrogen, chloride. The solvent removed *in vacuo*. The residue was crystallised from ethanol to obtain the title compound as the hydrochloride, m.p. 244—247°C dec.

Analysis for: $C_{20}H_{24}N_2O_3 . HCl$

| | |
|---|---|
| Calculated: | C, 63.73; H, 6.68; N, 7.43; Cl, 9.41 |
| Found: | C, 63.85; H, 6.77; N, 7.38; Cl, 9.33 |

P.R.=54 at $10^{-5}$ M
B.P.= −40 at 4 hours.

Example 18

4-[2-(n-Butyl)phenyl]-1,4,5,6,7,8-hexahydro-2-methyl-5-oxo-7-(phenylmethyl)-1,7-naphthyridine-3-carboxylic acid methyl ester

A mixture of 10.6 g. of 1-benzyl-3-oxo-5-hydroxy-1,2,3,6-tetrahydropyridine, 5.95 g. of methyl 3-aminocrotonate (97%), 8.1 g. of 2-(*n*-butyl)benzaldehyde and 125 ml. of ethanol was refluxed for 3 hours. The solvent was removed *in vacuo*. The viscous residue was crystallised by dissolving in ethanol and cooling in an ice bath. The solid was dissolved in methanol and saturated with hydrogen chloride. The solution was evaporated to a solid residue. Recrystallisation from 95% ethanol afforded the title compound as the hydrochloride, m.p. 220—223°C dec.

Analysis for: $C_{28}H_{32}N_2O_3$ . HCl

| | |
|---|---|
| Calculated: | C, 69.91; H, 6.91; N, 5.82; Cl, 7.37 |
| Found: | C, 69.42; H, 7.09; N, 5.61; Cl, 7.42 |

P.R.=61 at $10^{-6}$ M.

Example 19

4-[2-(n-Butyl)phenyl]-1,4,5,6,7,8-hexahydro-2-methyl-5-oxo-1,7-naphthyridine-3-carboxylic acid methyl ester

4-[2-(*n*-butyl)phenyl]-1,4,5,6,7,8-hexahydro-2-methyl-5-oxo-7-(phenylmethyl)-1,7-naphthyridine-3-carboxylic acid methyl ester hydrochloride (9.5 g), 0.5 g. of 10% palladium on carbon, 200 ml. of methanol and 50 ml. of water were shaken with hydrogen at an initial pressure of 2.76 bars (40 psi). After 2 hours, the catalyst was separated by filtration. The filtrate was evaporated *in vacuo* to a solid residue. Recrystallisation from ethanol afforded the title compound as the hydrochloride, m.p. 259—260°C.

Analysis for: $C_{21}H_{26}N_2O_3$ . HCl

| | |
|---|---|
| Calculated: | C, 64.52; H, 6.96; N, 7.16; Cl, 9.07 |
| Found: | C, 64.41; H, 7.01; N, 7.31; Cl, 9.18 |

P.R.=49.2 at $10^{-5}$ M
B.P.= −24 at 1.5 hours.

Example 20

4-(2,3-Dimethylphenyl)-1,4,5,6,7,8-hexahydro-2-methyl-5-oxo-7-(phenylmethyl)-1,7-naphthyridine-3-carboxylic acid methyl ester

A mixture of 16.2 g. of 1-benzyl-3-oxo-5-hydroxy-1,2,3,6-tetrahydropyridine, 9.2 g. of methyl 3-aminocrotonate, 11.6 g. of 2,3-dimethylbenzaldehyde and 180 ml. of ethanol was heated at reflux for 3 hours. The solvent was removed in vacuo and the residue was recrystallised from ethanol to obtain 14 g. of solid, m.p. 208—211°C. The solid was suspended in methanol and saturated with hydrogen chloride. The solvent was removed *in vacuo*. The residue was recrystallised from methanol to obtain the title compound as the hydrochloride, m.p. 238—239° d.

Analysis for: $C_{26}H_{28}N_2O_3$ . HCl

| | |
|---|---|
| Calculated: | C, 68.94; H, 6.45; N, 6.18; Cl, 7.83 |
| Found: | C, 68.78; H, 6.51; N, 6.41; Cl, 7.77 |

P.R.=33.3 at $10^{-6}$ M; 80.4 at $10^{-5}$ M
B.P.= −24 at 1.5 hours; −39 at 4 hours.

Example 21

4-(2,3-Dimethylphenyl)-1,4,5,6,7,8-hexahydro-2-methyl-5-oxo-1,7-naphthyridine-3-carboxylic acid methyl ester

Eight grams of the compound produced in Example 20, 0.5 g. of 10% palladium on carbon, 200 ml. of methanol, 50 ml. of water and 3 drops of concentrated hydrochloric acid were shaken with hydrogen at an initial pressure of 2.76 bars (40 p.s.i.). After two hours, the catalyst was separated by filtration. The filtrate was evaporated to dryness *in vacuo*. The residue was triturated with ethanol and separated to obtain the title compound as the hydrochloride, m.p. 254—255°C, dec.

Analysis for: $C_{19}H_{22}N_2O_3$ . HCl

| | |
|---|---|
| Calculated: | C, 62.89; H, 6.39; N, 7.72; Cl, 9.77 |
| Found: | C, 62.51; H, 6.58; N, 7.76; Cl, 9.46 |

P.R.=76.7 at $10^{-5}$ M
B.P.= −44 at 1.5 hours; −45 at 4 hours.

Example 22

1,4,5,6,7,8-Hexahydro-2-methyl-5-oxo-7-phenylmethyl-4-[2-(trifluoromethyl)phenyl]-1,7-naphthyridine-3-carboxylic acid, 2-(N-benzyl-N-methylamino)ethyl ester

A mixture of 15.2 g. of 1-benzyl-3-oxo-5-hydroxy-1,2,3,6-tetrahydropyridine, 13.1 g. of *o*-trifluoromethylbenzaldehyde, 18.7 g. of 2-(N-benzyl-N-methylamino)ethyl acetoacetate, 6 ml. of 28% ammonium hydroxide and 200 ml. of isopropanol was heated at reflux for 3 hours. The solution was evaporated to dryness. The residue was dissolved in 100 ml. of methylenechloride and extracted with 150 ml. of 15% hydrochloric acid. The acid extract was cooled and made basic with saturated sodium carbonate solution. The mixture was extracted with methylenechloride. The methylenechloride solution was dried over magnesium sulphate, then evaporated *in vacuo* to a solid residue. The residue was recrystallised from ethanol to obtain the title compound, m.p. 183—185°C.

Analysis for: $C_{34}H_{34}N_3F_3O_3$

| | |
|---|---|
| Calculated: | C, 69.25; H, 5.81; N, 7.12 |
| Found: | C, 68.90; H, 6.02; N, 7.27 |

P.R.=55.6 at $10^{-6}$ M
B.P.= −74 at 1.5 hours; −68 at 4 hours.

Example 23

1,4,5,6,7,8-Hexahydro-2,7-dimethyl-4-[2,3,4,5,6-pentafluorophenyl]-5-oxo-1,7-naphthyridine-3-carboxylic acid methyl ester

1-Methyl-3-oxo-5-hydroxy-1,2,3,6-tetrahydropyridine (6.36 g.), 5.92 g. of methyl 3-aminocrotonate, 9.8 g. of pentafluorobenzaldehyde and 125 ml. of methanol were refluxed for 4 hours. The reaction mixture was filtered and the filtrate was evaporated to dryness *in vacuo*. The residue was slurried with ethanol and filtered. Recrystallisation from methanol afforded the title compound; m.p. 250—253°C. dec.

Analysis for: $C_{18}H_{15}N_2F_5O_3$

| | |
|---|---|
| Calculated: | C, 53.74; H, 3.76; N, 6.96 |
| Found: | C, 53.42; H, 4.02; N, 6.85 |

Hydrochloride, m.p. 240—243°C. dec. (recrystallised from methanol).

Analysis for: $C_{18}H_{15}N_2F_5O_3 \cdot HCl$

| | |
|---|---|
| Calculated: | C, 49.27; H, 3.68; N, 6.38; Cl, 8.08. |
| Found: | C, 49.12; H, 3.59; N, 6.38; Cl, 8.07 |

P.R.=22.9 ($10^{-5}$ M)
B.P.=50 mg/kg −95 at 1.5 hours; −89 at 4 hours.
25 mg/kg −71 at 1.5 hours; −49 at 4 hours.
10 mg/kg −63 at 1.5 hours; −31 at 4 hours.
5 mg/kg −21 at 1.5 hours; −10 at 4 hours.

Example 24

1,4,5,6,7,8-Hexahydro-2-methyl-5-oxo-4-(2,3,4,5,6-pentafluorophenyl)-7-(phenylmethyl)-1,7-naphthyridine-3-carboxylic acid methyl ester

A mixture of 10.2 g. of 1-benzyl-3-oxo-5-hydroxy-1,2,3,6-tetrahydropyridine, 5.93 g. of methyl 3-aminocrotonate, 10 g. of pentafluorobenzaldehyde and 125 ml. of methanol was refluxed for 4 hours. The solution was allowed to cool to room temperature. The solid was separated by filtration. The solid, m.p. 258—260°C. dec., was suspended in methanol and saturated with hydrogen chloride. The solution was evaporated to dryness *in vacuo*. Recrystallisation of the residue from methanol afforded the title compound as the hydrochloride, m.p. 198—200°C. dec.

Analysis for: $C_{24}H_{19}N_2F_5O_3 \cdot HCl$

| | |
|---|---|
| Calculated: | C, 55.97; H, 3.91; N, 5.44; Cl, 6.88 |
| Found: | C, 55.53; H, 4.12; N, 5.30; Cl, 7.08 |

P.R.=56 ($10^{-6}$ M)
B.P.=50 mg/kg −20 at 1.5 hours; −23 at 4 hours.

Example 25

1,4,5,6,7,8-Hexahydro-2-methyl-5-oxo-4-(2,3,4,5,6-pentafluorophenyl)-1,7-naphthyridine-3-carboxylic acid methyl ester

Six grams of 1,4,5,6,7,8-hexahydro-2-methyl-5-oxo-4-(2,3,4,5,6-pentafluorophenyl)-7-(phenylmethyl)-1,7-naphthyridine-3-carboxylic acid methyl ester hydrochloride (Example 24), 200 ml. of methanol, 15 ml. of water and 0.5 g. of 10% palladium on carbon were shaken with hydrogen at an initial pressure of 3.45 bars (50 psi). After 3 hours, the catalyst was separated and the filtrate was evaporated to dryness *in vacuo*. The residue was slurried with ethanol and filtered. Recrystallisation from methanoldiethyl ether afforded the title compound as the hydrochloride, m.p. 268—270°C. dec.

Analysis for: $C_{17}H_{13}N_2F_5O_3 . HCl$

| | |
|---|---|
| Calculated: | C, 48.07; H, 3.32; N, 6.59; Cl, 8.34 |
| Found: | C, 48.36; H, 3.53; N, 6.69; Cl, 7.94 |

P.R.=65.2 ($10^{-6}$ M); 78.7 ($10^{-5}$ M).
B.P.=50 mg/kg −64 at 1.5 hours; −88 at 4 hours.
25 mg/kg −76 at 1.5 hours
10 mg/kg −79 at 1.5 hours; −24 at 4 hours.
5 mg/kg −48 at 1.5 hours; −36 at 4 hours.
2.5 mg/kg −34 at 1.5 hours; −30 at 4 hours.

**Claims for the Contracting States: BE, CH, DE, FR, IT, LI, LU, NL, SE**

1. A compound of the formula

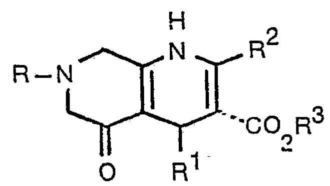

(I)

in which R is hydrogen, alkyl of 1 to 6 carbon atoms or benzyl; $R^2$ is alkyl of 1 to 6 carbon atoms; $R^3$ is alkyl of 1 to 6 carbon atoms, alkoxyalkyl in which each alkyl moiety has 1 to 6 carbon atoms, $-CH_2CF_3$, $-CH_2CH_2CF_3$ or

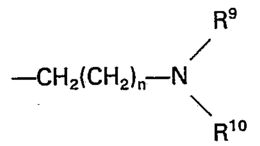

(II)

where $R^9$ is hydrogen or alkyl of 1 to 6 carbon atoms; and $R^{10}$ is alkyl of 1 to 6 carbon atoms or arylalkyl of 7 to 10 carbon atoms or $R^9$ and $R^{10}$ taken with the nitrogen atom to which they are attached form a pyrrolidinyl, imidazolidinyl, piperidyl, piperazinyl, 4-alkylpiperazinyl in which the alkyl group contains from 1 to 6 carbon atoms or morpholinyl heterocycle; and n is one of the integers 0, 1 or 2; $R^1$ is pentaafluorophenyl, tetrafluorophenyl or

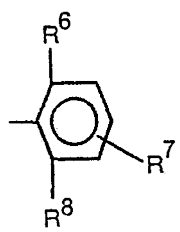

(III)

wherein $R^6$ and $R^8$ are, independently, hydrogen, alkyl of 1 to 6 carbon atoms, halo, trifluoromethyl, alkoxy of 1 to 6 carbon atoms, alkylthio of 1 to 6 carbon atoms, cyano or nitro; $R^7$ is hydrogen, alkyl of 1 to 6 carbon atoms, halo, trifluoromethyl, cyano or nitro; or $R^8$ is as defined above whilst $R^6$ and $R^7$ are in the ortho position to each other and taken together are butadienylene, tetramethylene or trimethylene; or a pharmaceutically acceptable salt thereof.

2. A compound as claimed in Claim 1, wherein $R^1$ is pentafluorophenyl or a group having the formula

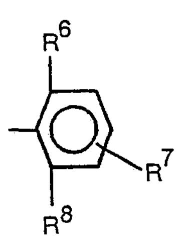

(III)

wherein $R^6$ and $R^7$ are as defined in Claim 1 and $R^8$ is hydrogen.

3. A compound as claimed in Claim 1 or 2 wherein $R^3$ is alkyl of 1 to 6 carbon atoms or

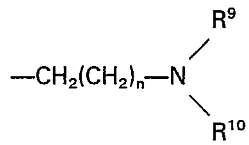

(II)

wherein n is 1 or 2, $R^{10}$ is benzyl or phenethyl and $R^9$ is as defined in Claim 1.

4. A compound selected from 1,4,5,6,7,8-hexahydro-2,7-dimethyl-4-[2,3,4,5,6-pentafluorophenyl]-5-oxo-1,7-naphthyridine-3-carboxylic acid methyl ester; 1,4,5,6,7,8-hexahydro-2-methyl-5-oxo-4-(2,3,4,5,6-pentafluorophenyl)-7-(phenylmethyl)-1,7-naphthyridine-3-carboxylic acid methyl ester and their pharmaceutically acceptable salts.

5. 1,4,5,6,7,8-Hexahydro-2-methyl-5-oxo-4-(2,3,4,5,6-pentafluorophenyl)-1,7-naphthyridine-3-carboxylic acid methyl ester or a pharmaceutically acceptable salt thereof.

6. A compound selected from 1,4,5,6,7,8-hexahydro-2-methyl-5-oxo-4-[2-(trifluoromethyl)-phenyl]-1,7-naphthyridine-3-carboxylic acid methyl ester; its 7-(methyl or benzyl) substitution product; 1,4,5,6,7,8-hexahydro-2-methyl-5-oxo-7-phenylmethyl-4-[2-(trifluoromethyl)phenyl] - 1,7-naphthyridine-3-carboxylic acid 2-(N-benzyl-N-methylamino)ethyl ester and their pharmaceutically acceptable salts.

7. A compound selected from 1,4,5,6,7,8-hexahydro-2-methyl-4-(2-methylphenyl)-5-oxo-1,7-naphthyridine-3-carboxylic acid methyl ester, the 7-(methyl or benzyl) substitution product thereof and their pharmaceutically acceptable salts.

8. 4-Aryl-1,4,5,6,7,8-hexahydro-2-methyl-5-oxo-1,7-naphthyridine-3-carboxylic acid methyl ester (wherein the aryl group is 3-nitrophenyl, 3-(trifluoromethyl)phenyl, 2-fluorophenyl, 2-(n-butyl)phenyl or 2,3-dimethylphenyl); or 4-aryl-1,4,5,6,7,8-hexahydro-2,7-dimethyl-5-oxo-1,7-naphthyridine-3-carboxylic acid methyl ester (wherein the aryl group is 2-nitrophenyl, 3-nitrophenyl, 2-chlorophenyl, 2,6-dichlorophenyl, 2,5-dimethylphenyl, 3-cyclophenyl or 2,3-dimethylphenyl); or 4-aryl-1,4,5,6,7,8-hexahydro-2-methyl-5-oxo-7-(phenylmethyl)-1,7-naphthyridine-3-carboxylic acid methyl ester (wherein the aryl group is 2-(n-butyl)phenyl, 2,3-dimethylphenyl, 2-fluorophenyl-3-(trifluoromethyl)phenyl or 3-nitrophenyl); or a pharmaceutically acceptable salt thereof.

9. A compound as claimed in any one of claims 1 to 8 for use as an antihypertensive agent.

10. A pharmaceutical composition comprising a compound as claimed in any one of claims 1 to 8 in association with a pharmaceutically acceptable carrier.

11. A pharmaceutical composition as claimed in claim 10 in the form of a tablet of capsule.

12. A process for the preparation of a compound having the formula

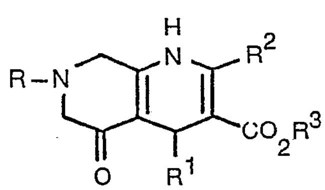

(I)

(wherein R, $R^1$, $R^2$ and $R^3$ are as defined in claim 1) or a pharmaceutically acceptable salt thereof, comprising

(a) reacting a compound having formula IV

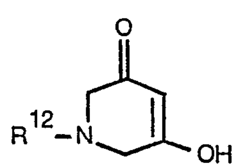

(IV)

(wherein $R^{12}$ is alkyl of 1 to 6 carbon atoms or benzyl), an aldehyde having formula $R^1CHO$ (wherein $R^1$ is as defined above) and a substituted or unsubstituted 3-aminocrotonate ester having formula VI

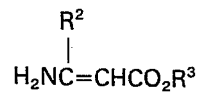

(VI)

(wherein $R^2$ and $R^3$ are as defined above); or

(b) reacting a compound having formula VIII

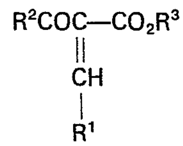

(VIII)

(wherein $R^1$, $R^2$ and $R^3$ are as defined above) with a compound having the formula IV

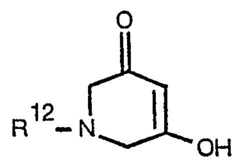

(IV)

(wherein $R^{12}$ is as defined above) in the presence of ammonia; or

(c) subjecting a compound having formula VII

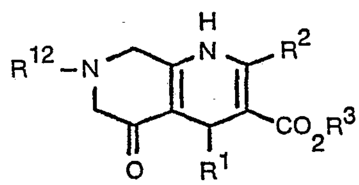

(VII)

(wherein $R^1$, $R^2$ and $R^3$ are as defined above and $R^{12}$ is benzyl) or a salt thereof to hydrogenolysis; and, if desired, forming a free base form of the compound having formula I by neutralising an acid addition salt thereof with a base or forming a pharmaceutically acceptable salt by neutralising the free base with an acid or by subjecting another salt to metathetic displacement.

13. A process as claimed in claim 12, wherein step (a) is carried out and the substituted or unsubstituted 3-aminocrotonate ester is prepared *in situ* by ammonolysis of the alkanoylacetic acid ester having the formula $R^2COCH_2CO_2R^3$ (in which $R^2$ and $R^3$ are as defined in claim 1).

**Claims for the Contracting State: AT**

1. A process for the preparation of a compound of the formula

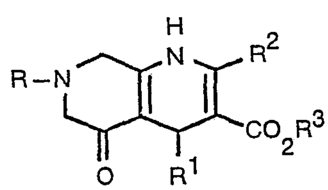

(I)

in which

R is hydrogen, alkyl of 1 to 6 carbon atoms or benzyl;

$R^2$ is alkyl of 1 to 6 carbon atoms;

$R^3$ is alkyl of 1 to 6 carbon atoms, alkoxyalkyl in which each alkyl moiety has 1 to 6 carbon atoms, $—CH_2CF_3$, $—CH_2CH_2CF_3$ or

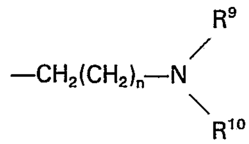 (II)

where $R^9$ is hydrogen or alkyl of 1 to 6 carbon atoms; and

$R^{10}$ is alkyl of 1 to 6 carbon atoms or arylalkyl or 7 to 10 carbon atoms or $R^9$ and $R^{10}$ taken with the nitrogen atom to which they are attached form a pyrrolidinyl, imidazolidinyl, piperidyl, piperazinyl, 4-alkylpiperazinyl in which the alkyl group contains from 1 to 6 carbon atoms or morpholinyl heterocycle; and

n is one of the integers 0, 1 or 2;

$R^1$ is pentafluorophenyl, tetrafluorophenyl or

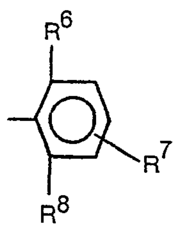 (III)

wherein

$R^6$ and $R^8$ are, independently, hydrogen, alkyl or 1 to 6 carbon atoms, halo, trifluoromethyl alkoxy of 1 to 6 carbon atoms, alkylthio of 1 to 6 carbon atoms, cyano or nitro;

$R^7$ is hydrogen, alkyl or 1 to 6 carbon atoms, halo, trifluoromethyl, cyano or nitro; or

$R^8$ is as defined above whilst $R^6$ and $R^7$ are in the ortho position to each other and taken together are butadienylene, tetramethylene or trimethylene; or a pharmaceutically acceptable salt thereof, characterised by

(a) reacting a compound having formula IV

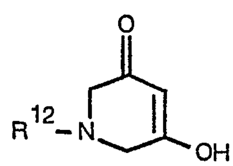 (IV)

(wherein $R^{12}$ is alkyl of 1 to 6 carbon atoms or benzyl), an aldehyde having formula $R^1CHO$ (wherein $R^1$ is as defined above) and a substituted or unsubstituted 3-aminocrotonate ester having formula VI

$R^2$
|
$H_2NC{=}CHCO_2R^3$ (VI)

(wherein $R^2$ and $R^3$ are as defined above); or

(b) reacting a compound having formula VIII

$R^2COC—CO_2R^3$
||
CH
|
$R^1$ (VIII)

(wherein $R^1$, $R^2$ and $R^3$ are as defined above) with a compound having the formula IV

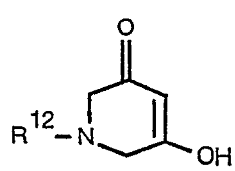 (IV)

(wherein $R^{12}$ is as defined above) in the presence of ammonia; or

(c) subjecting a compound having formula

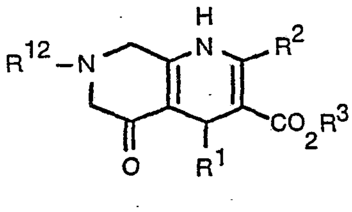

(VII)

(wherein $R^1$, $R^2$ and $R^3$ are as defined above and $R^{12}$ is benzyl) or a salt thereof to hydrogenolysis; and, if desired, forming a free base form of the compound having formula I by neutralising an acid addition salt thereof with a base or forming a pharmaceutically acceptable salt by neutralising the free base with an acid or by subjecting another salt to metathetic displacement.

2. A process as claimed in claim 1, wherein step (a) is carried out and the substituted or unsubstituted 3-aminocrotonate ester is prepared *in situ* by ammonolysis of the alkanoylacetic acid ester having the formula $R^2COCH_2CO_2R^3$ (in which $R^2$ and $R^3$ are as defined in claim 1).

3. A process for the preparation of a compound having formula I

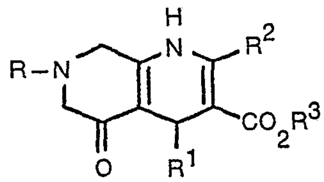

(I)

(wherein R, $R^1$, $R^2$ and $R^3$ are as defined in claim 1), characterised in that an acid addition salt thereof is neutralised with a base.

4. A process for the preparation of a pharmaceutically acceptable salt of a compound having formula I

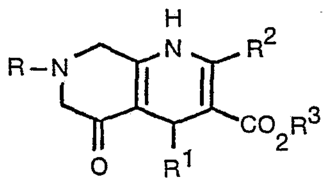

(I)

(wherein R, $R^1$, $R^2$ and $R^3$ are as defined in claim 1), characterised in that

(a) a compound having formula I as defined above is neutralised with an acid; or

(b) another salt of the compound having formula I is subjected to metathetic displacement.

5. A process according to claim 4, characterised in that $R^1$ is tetrafluorophenyl.

6. A process according to claim 4, characterised in that $R^1$ is

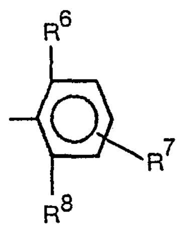

(III)

wherein $R^6$, $R^7$, and $R^8$ are as defined in claim 1.

7. A process for the preparation of a compound having the formula I

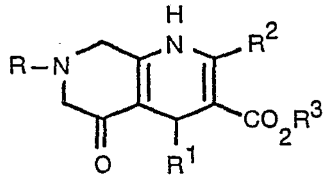

(I)

(wherein $R^1$ is pentafluorophenyl and R, $R^2$ and $R^3$ are as defined in claim 1) or a pharmaceutically acceptable salt thereof characterised by

(a) subjecting a compound having the formula XII

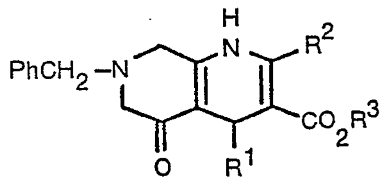

(XII)

(wherein $PhCH_2$ is benzyl and $R^1$, $R^2$ and $R^3$ are as defined above) or a salt thereof to hydrogenolysis; or

(b) reacting together a compound having the formula IV

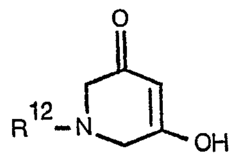

(IV)

(wherein $R^{12}$ is alkyl of 1 to 6 carbon atoms or benzyl), an aldehyde having the formula $R^1CHO$ (wherein $R^1$ is as defined above) and a substituted or unsubstituted 3-aminocrotonate ester having the formula VI

$$NH_2\overset{\overset{\large R^2}{|}}{C}=CHCO_2R^3 \qquad (VI)$$

(wherein $R^2$ and $R^3$ are as defined above) and, if desired, converting a compound having formula I into a pharmaceutically acceptable salt thereof by neutralization with an acid.

8. A process for the preparation of a compound having the formula I

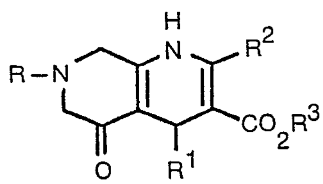

(I)

(wherein R, $R^2$ and $R^3$ are as defined in claim 1 and $R^1$ is a group having the formula

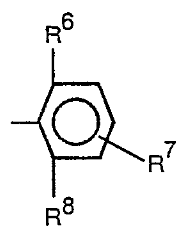

wherein $R^6$, $R^7$ and $R^8$ are as defined in claim 1) or a pharmaceutically acceptable salt thereof, characterised by

(a) subjecting a compound having the formula XII

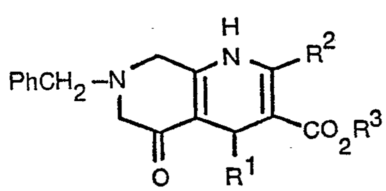

(XII)

(wherein $PhCH_2$ is benzyl and $R^1$, $R^2$ and $R^3$ are as defined above) or a salt thereof to hydrogenolysis; or

(b) reacting together a compound having the formula IV

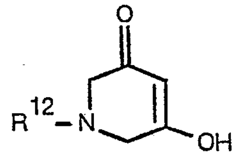

(IV)

(wherein $R^{12}$ is alkyl of 1 to 6 carbon atoms or benzyl), an aldehyde having the formula $R^1CHO$ (wherein $R^1$ is as defined above) and a substituted or unsubstituted 3-aminocrotonate ester having the formula VI

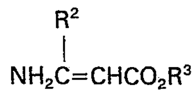

(VI)

(wherein $R^2$ and $R^3$ are as defined above) and, if desired, converting a compound having formula I into a pharmaceutically acceptable salt thereof by neutralisation with an acid.

9. A process for the preparation of a pharmaceutical composition characterised by bringing a compound having the formula I

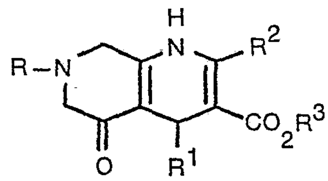

(I)

(wherein R, $R^1$, $R^2$ and $R^3$ are as defined in claim 1) into association with a pharmaceutically acceptable carrier.

10. A process according to claim 9, characterised in that the composition is prepared in the form of a tablet or capsule.

11. A process according to claim 9 or 10, wherein $R^1$ is a group having the formula

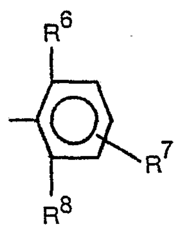

(III)

wherein $R^6$, $R^7$ and $R^8$ are as defined in claim 1.

12. A process according to claim 9 or 10, wherein $R^1$ is pentafluorophenyl.

**Patentansprüche für die Vertragsstaaten BE CH DE FR IT LI LU NL SE:**

1. Eine Verbindung der Formel

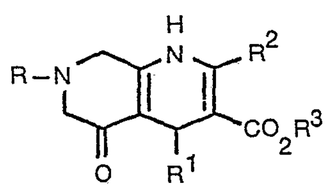

(I)

worin R Wasserstoff, Alkyl mit 1 bis 6 C-Atomen oder Benzyl bedeutet; $R^2$ Alkyl mit 1 bis 6 C-Atomen ist; $R^3$ Alkyl mit 1 bis 6 C-Atomen, Alkoxyalkyl, wobei jede Alkylgruppe 1 bis 6 C-Atome aufweist, $—CH_2CF_3$, $—CH_2CH_2CF_3$ oder

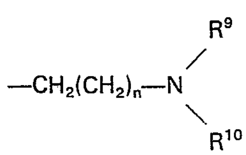

(II) ist,

wobei $R^9$ Wasserstoff oder Alkyl mit 1 bis 6 C-Atomen ist und $R^{10}$ Alkyl mit 1 bis 6 C-Atomen oder Arylalkyl mit 7 bis 10 C-Atomen darstellt oder $R^9$ und $R^{10}$ zusammen mit dem Stickstoffatom, an das sie gebunden sind, einen Pyrrolidinyl-, Imidazolidinyl-, Piperidyl-, Piperazinyl-, 4-Alkylpiperazinyl-, wobei die Alkylgruppe 1 bis 6 C-Atome enthält, oder einen Morpholinylheterocyclus bilden, und n eine der ganzen Zahlen Null, 1 oder 2 ist; $R^1$ Pentafluorphenyl, Tetrafluorphenyl oder

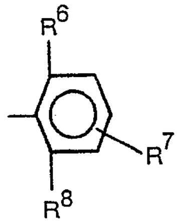

(III)

darstellt, wobei $R^6$ und $R^8$ unabhängig voneinander Wasserstoff, Alkyl mit 1 bis 6 C-Atomen, Halogen, Trifluormethyl, Alkoxy mit 1 bis 6 C-Atomen, Alkylthio mit 1 bis 6 C-Atomen, Cyano oder Nitro sind; $R^7$ Wasserstoff, Alkyl mit 1 bis 6 C-Atomen, Halogen, Trifluormethyl, Cyano oder Nitro bedeutet oder $R^8$ wie oben definiert ist, während $R^6$ und $R^7$ sich zueinander in o-Stellung befinden und miteinander Butadienylen, Tetramethylen oder Trimethylen bedeuten; oder ein pharmazeutisch annehmbares Salz hievon.

2. Eine Verbindung wie in Anspruch 1 beanspruch, worin $R^1$ Pentafluorphenyl oder eine Gruppe der Formel

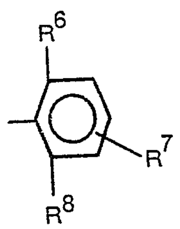

(III)

ist, wobei $R^6$ und $R^7$ wie in Anspruch 1 definiert sind und $R^8$ Wasserstoff ist.

3. Eine Verbindung wie in Anspruch 1 oder 2 beansprucht, worin $R^3$ Alkyl mit 1 bis 6 C-Atomen oder

$$-CH_2(CH_2)_n-N\begin{matrix}/R^9\\ \backslash R^{10}\end{matrix} \qquad (II)$$

ist, wobei n 1 oder 2 ist, $R^{10}$ Benzyl oder Phenäthyl bedeutet und $R^9$ wie in Anspruch 1 definiert ist.

4. Eine Verbindung ausgewählt unter 1,4,5,6,7,8-Hexahydro-2,7-dimethyl-4-[2,3,4,5,6-pentafluorphenyl]-5-oxo-1,7-naphthridin-3-carbonsäure-methylester; 1,4,5,6,7,8-Hexahydro-2-methyl-3-oxo-4-(2,3,4,5,6-pentafluorphenyl)-7-(phenylmethyl)-1,7-naphthyridin-3-carbonsäure-methylester und ihren pharmazeutisch annehmbaren Salzen.

5. 1,4,5,6,7,8-Hexahydro-2-methyl-5-oxo-4-(2,3,4,5,6-pentafluorphenyl)-1,7-naphthyridine-3-carbonsäure-methylester oder ein pharmazeutisch annehmbares Salz hievon.

6. Eine Verbindung ausgewählt unter 1,4,5,6,7,8-Hexahydro-2-methyl-5-oxo-4-[2-(trifluormethyl)-phenyl]-1,7-naphthyridin-3-carbonsäure-methylester; dessen 7-Methyl oder Benzyl)-Substitutionsprodukt; 1,4,5,6,7,8-Hexahydro-2-methyl-5-oxo-7-phenylmethyl-4-[2-(trifluormethyl)-phenyl]-1,7-naphthyridin-3-carbonsäure-2-(N-benzyl-N-methylamino)-äthylester und deren pharmazeutisch annehmbaren Salzen.

7. Eine Verbindung ausgewählt unter 1,4,5,6,7,8-Hexahydro-2-methyl-4-(2-methylphenyl)-5-oxo-1,7-naphthyridin-3-carbonsäure-methylester, dem 7-(Methyl- oder Benzyl)-Substitutionsprodukt hievon und deren pharmazeutisch annehmbaren Salzen.

8. 4-Aryl-1,4,5,6,7,8-hexahydro-2-methyl-5-oxo-1,7-naphthyridin-3-carbonsäure-methylester (worin die Arylgruppe 3-Nitrophenyl, 3-(Trifluormethyl)-phenyl, 2-Fluorphenyl, 2-(n-Butylphenyl oder 2,3-Dimethylphenyl ist); oder 4-Aryl-1,4,5,6,7,8-hexahydro-2,7-dimethyl-5-oxo-1,7-naphthyridin-3-carbonsäure-methylester (worin die Arylgruppe 2-Nitrophenyl, 3-Nitrophenyl, 2-Chlorphenyl, 2,6-Dichlorphenyl, 2,5-Dimethylphenyl, 3-Cyanophenyl oder 2,3-Dimethylphenyl ist); oder 4-Aryl-1,4,5,6,7,8-hexahydro-2-methyl-3-oxo-7-(phenylmethyl)-1,7-naphthyridin-3-carbonsäure-methylester (worin die Arylgruppe 2-(n-Butyl)-phenyl, 2,3-Dimethylphenyl, 2-Fluorphenyl, 3-(Trifluormethyl)-phenyl oder 3-Nitrophenyl ist); oder ein pharmazeutisch annehmbares Salz hievon.

9. Eine Verbindung wie in einem der Ansprüche 1 bis 8 beansprucht zur Verwendung als antihypertonisches Mittel.

10. Eine pharmazeutische Zusammensetzung umfassend eine Verbindung wie in einem der Ansprüche 1 bis 8 beansprucht in Vereinigung mit einem pharmazeutisch annehmbaren Träger.

11. Eine pharmazeutische Zusammensetzung wie in Anspruch 10 beanspruch in Form einer Tablette oder Kapsel.

12. Verfahren zur Herstellung einer Verbindung der Formel

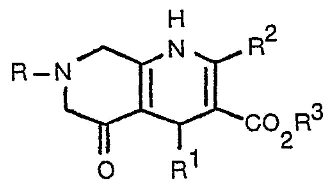

(I)

(worin R, $R^1$, $R^2$ und $R^3$ wie in Anspruch 1 definiert sind) oder eines pharmazeutisch annehmbaren Salzes hievon, umfassend

(a) das Umsetzen einer Verbindung der Formel (IV)

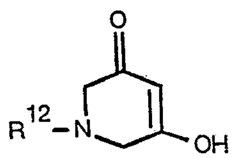

(IV)

(worin $R^{12}$ Alkyl mit 1 bis 6 C-Atomen oder Benzyl ist) eines Aldehyds der Formel $R^1CHO$ (worin $R^1$ wie oben definiert ist) und eines substituierten oder unsubstituierten 3-Aminocrotonatesters der Formel

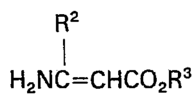

(VI)

(worin $R^2$ und $R^3$ wie oben definiert sind); oder

(b) das Umsetzen einer Verbindung der Formel (VIII)

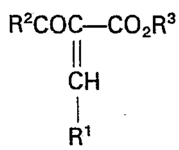

(VIII)

(worin $R^1$, $R^2$ und $R^3$ wie oben definiert sind) mit einer Verbindung der Formel (IV)

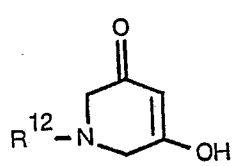

(IV)

(worin $R^{12}$ wie oben definiert ist) in Anwesenheit von Ammoniak; oder

(c) das Unterwerfen einer Verbindung der Formel (VII)

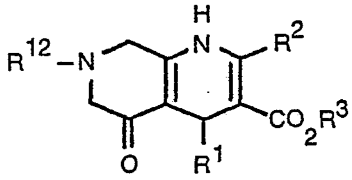

(VII)

(worin $R^1$, $R^2$ und $R^3$ wie oben definiert sind und $R^{12}$ Benzyl ist) oder eines Salzes hievon der Hydrogenolyse; und, wenn gewünscht, Bilden einer freien Basenform der Verbindung der Formel (I) durch Neutralisieren eines Säureadditionssalzes hievon mit einer Base oder Bilden eines pharmazeutisch annehmbaren Salzes durch Neutralisieren der freien Base mit einer Säure oder Unterwerfen eines anderen Salzes der metathetischen Verdrängung.

13. Verfahren wie in Anspruch 12 beansprucht, worin Schritt (a) durchgeführt wird und der substituierte oder unsubstituierte 3-Aminocrotonatester in situ durch Ammonolyse des Alkanoylessigsäureesters der Formel $R^2COCH_2CO_2R^3$ (worin $R^2$ und $R^3$ wie in Anspruch 1 definiert sind) hergestellt wird.

**Patentansprüche für den Vertragsstaat AT:**

1. Verfahren zur Herstellung einer Verbindung der Formel

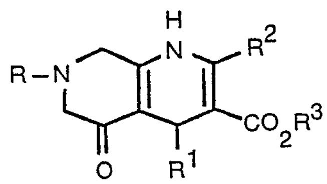

(I)

worin R Wasserstoff, Alkyl mit 1 bis 6 C-Atomen oder Benzyl bedeutet; $R^2$ Alkyl mit 1 bis 6 C-Atomen ist; $R^3$ Alkyl mit 1 bis 6 C-Atomen, Alkoxyalkyl, wobei jede Alkylgruppe 1 bis 6 C-Atome aufweist, $—CH_2CF_3$, $—CH_2CH_2CF_3$ oder

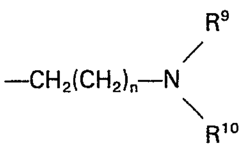

(II) ist,

wobei $R^9$ Wasserstoff oder Alkyl mit 1 bis 6 C-Atomen ist und $R^{10}$ Alkyl mit 1 bis 6 C-Atomen oder Arylalkyl mit 7 bis 10 C-Atomen darstellt oder $R^9$ und $R^{10}$ zusammen mit dem Stickstoffatom, an das sie gebunden sind, einen Pyrrolidinyl-, Imidazolidinyl-, Piperidyl-, Piperazinyl-, 4-Alkylpiperazinyl-, wobei die Alkylgruppe 1 bis 6 C-Atome enthält, oder einen Morpholinylheterocyclus bilden, und n eine der ganzen Zahlen Null, 1 oder 2 ist; $R^1$ Pentafluorphenyl, Tetrafluorphenyl oder

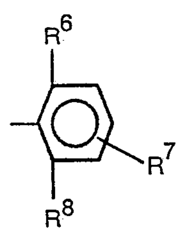

(III)

darstellt, wobei $R^6$ und $R^8$ unabhängig voneinander Wasserstoff, Alkyl mit 1 bis 6 C-Atomen, Halogen, Trifluormethyl, Alkoxy mit 1 bis 6 C-Atomen, Alkylthio mit 1 bis 6 C-Atomen, Cyano oder Nitro sind; $R^7$ Wasserstoff, Alkyl mit 1 bis 6 C-Atomen, Halogen, Trifluormethyl, Cyano oder Nitro bedeutet oder $R^8$ wie oben definiert ist, während $R^6$ und $R^7$ sich zueinander in o-Stellung befinden und miteinander Butadienylen, Tetramethylen oder Trimethylen bedeuten, oder eines pharmazeutisch annehmbaren Salzes hievon, gekennzeichnet durch

(a) das Umsetzen einer Verbindung der Formel (IV)

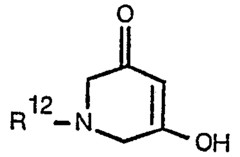

(IV)

(worin $R^{12}$ Alkyl mit 1 bis 6 C-Atomen oder Benzyl ist) eines Aldehyds der Formel $R^1CHO$ (worin $R^1$ wie oben definiert ist) und eines substituierten oder unsubstituierten 3-Aminocrotonatesters der Formel

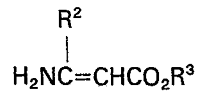

(VI)

(worin $R^2$ und $R^3$ wie oben definiert sind); oder

(b) das Umsetzen einer Verbindung der Formel (VIII)

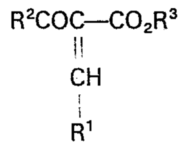

(VIII)

(worin $R^1$, $R^2$ und $R^3$ wie oben definiert sind) mit einer Verbindung der Formel (IV)

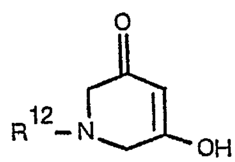

(IV)

(worin $R^{12}$ wie oben definiert ist) in Anwesenheit von Ammoniak; oder

(c) das Unterwerfen einer Verbindung der Formel (VII)

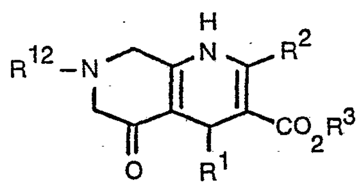

(VII)

(worin $R^1$, $R^2$ und $R^3$ wie oben definiert sind und $R^{12}$ Benzyl ist) oder eines Salzes hievon der Hydrogenolyse; und, wenn gewünscht, Bilden einer freien Basenform der Verbindung der Formel (I) durch Neutralisieren eines Säureadditionssalzes hievon mit einer Base oder Bilden eines pharmazeutisch annehmbaren Salzes durch Neutralisieren der freien Base mit einer Säure oder Unterwerfen eines anderen Salzes der metathetischen Verdrängung.

2. Verfahren wie in Anspruch 1 beansprucht, worin Schritt (a) durchgeführt wird und der substituierte oder unsubstituierte 3-Aminocrotonatester in situ durch Ammonolyse des Alkanoylessigsäureesters der Formel $R^2COCH_2CO_2R^3$ (worin $R^2$ und $R^3$ wie in Anspruch 1 definiert sind) hergestellt wird.

3. Verfahren zur Herstellung einer Verbindung der Formel (I)

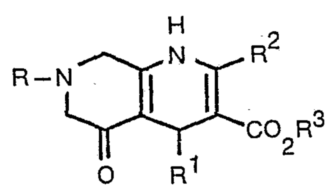

(I)

(worin R, $R^1$, $R^2$ und $R^3$ wie in Anspruch 1 definiert sind), dadurch gekennzeichnet, daß ein Säureadditionssalz hievon mit einer Base neutralisiert wird.

4. Verfahren zur Herstellung eines pharmazeutisch annehmbaren Salzes einer Verbindung der Formel (I)

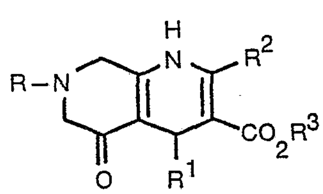

(I)

(worin R, $R^1$, $R^2$ und $R^3$ wie in Anspruch 1 definiert sind), dadurch gekennzeichnet, daß

(a) eine Verbindung der Formel (I), wie oben definiert, mit einer Säure neutralisiert wird oder

(b) ein anderes Salz der Verbindung der Formel (I) metathetischer Verdrängung unterworfen wird.

5. Verfahren nach Anspruch 4, dadurch gekennzeichnet, daß $R^1$ Tetrafluorphenyl ist.

6. Verfahren nach Anspruch 4, dadurch gekennzeichnet, daß $R^1$

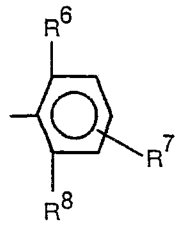

(III)

ist, wobei $R^6$, $R^7$ und $R^8$ wie in Anspruch 1 definiert sind.

7. Verfahren zur Herstellung einer Verbindung der Formel (I)

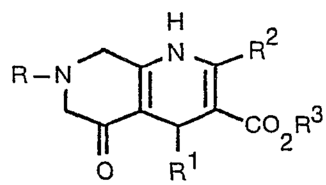

(I)

(worin $R^1$ Pentafluorphenyl ist und R, $R^2$ und $R^3$ wie in Anspruch 1 definiert sind) oder eines pharmazeutisch annehmbaren Salzes hievon, gekennzeichnet durch

(a) das Unterwerfen einer Verbindung der Formel (XII)

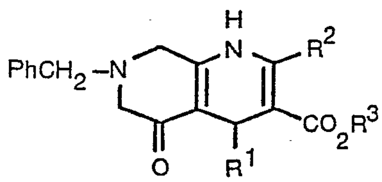

(XII)

(worin $PhCH_2$ Benzyl ist und $R^1$, $R^2$ und $R^3$ wie oben definiert sind) oder eines Salzes hievon der Hydrogenolyse; oder

(b) das Umsetzen einer Verbindung der Formel (IV)

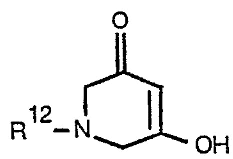

(IV)

(worin $R^{12}$ Alkyl mit 1 bis 6 C-Atomen oder Benzyl ist), eines Aldehyds der Formel $R^1CHO$ (worin $R^1$ wie oben definiert ist) und eines substituierten oder unsubstituierten 3-Aminocrotonatesters der Formel (VI)

$$NH_2\overset{\overset{\displaystyle R^2}{|}}{C}=CHCO_2R^3 \qquad \text{(VI)}$$

(worin $R^2$ und $R^3$ wie oben definiert sind) und, wenn gewünscht, Überführen einer Verbindung der Formel (I) in ein pharmazeutisch annehmbares Salz hievon durch Neutralisation mit einer Säure.

8. Verfahren zur Herstellung einer Verbindung der Formel (I)

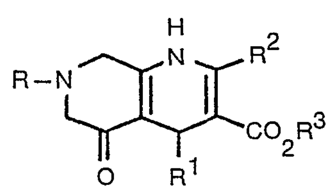

(I)

(worin R, $R^2$ und $R^3$ wie in Anspruch 1 definiert sind und $R^1$ eine Gruppe der Formel

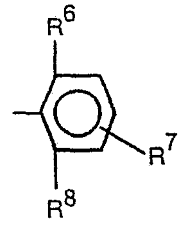

ist, wobei $R^6$, $R^7$ und $R^8$ wie in Anspruch 1 definiert sind) oder eines pharmazeutisch annehmbaren Salzes hievon, gekennzeichnet durch

(a) Unterwerfen einer Verbindung der Formel (XII)

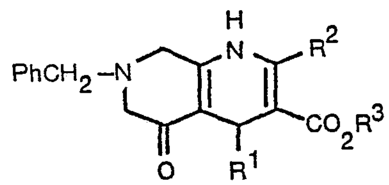

(XII)

(worin $PhCH_2$ Benzyl ist und $R^1$, $R^2$ und $R^3$ wie oben definiert sind) oder eines Salzes hievon der Hydrogenolyse; oder

(b) das Umsetzen einer Verbindung der Formel (IV)

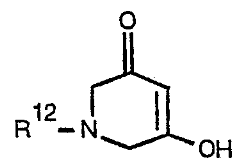

(IV)

(worin $R^{12}$ Alkyl mit 1 bis 6 C-Atomen oder Benzyl ist), eines Aldehyds der Formel $R^1CHO$ (worin $R^1$ wie oben definiert ist) und eines substituierten oder unsubstituierten 3-Aminocrotonatesters der Formel (VI)

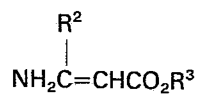

(VI)

(worin $R^2$ und $R^3$ wie oben definiert sind) und, wenn gewünscht, Überführen einer Verbindung der Formel (I) in ein pharmazeutisch annehmbares Salz hievon durch Neutralisation mit einer Säure.

9. Verfahren zur Herstellung einer pharmazeutischen Zusammensetzung, gekennzeichnet durch Vereinigen einer Verbindung der Formel (I)

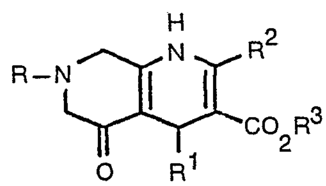

(I)

(worin R, $R^1$, $R^2$ und $R^3$ wie in Anspruch 1 definiert sind) mit einem pharmazeutisch annehmbaren Träger.

10. Verfahren nach Anspruch 9, dadurch gekennzeichnet, daß die Zusammensetzung in Form einer Tablette oder einer Kapsel hergestellt wird.

11. Verfahren nach Anspruch 9 oder 10, worin $R^1$ eine Gruppe der Formel

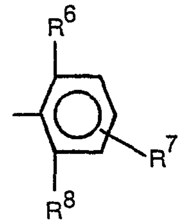

(III)

ist, wobei $R^6$, $R^7$ und $R^8$ wie in Anspruch 1 definiert sind.

12. Verfahren nach Anspruch 9 oder 10, worin $R^1$ Pentafluorphenyl ist.

**Revendications pour les Etats Contractants BE, CH, DE, FR, IT, LI, LU, NL, SE**

1. Composé de formule:

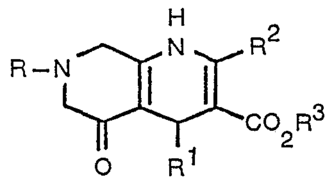

(I)

où R représente un atome d'hydrogène ou radical alcoyle de 1 à 6 atomes de carbone ou benzyle;

$R^2$ représente un radical alcoyle de 1 à 6 atomes de carbone;

$R^3$ représente un radical alcoyle de 1 à 6 atomes de carbone, alcoxyalcoyle dont chaque partie alcoyle compte 1 à 6 atomes de carbone, $—CH_2CF_3$, $—CH_2CH_2CF_3$ ou

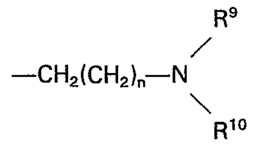

(II)

où $R^9$ représente un atome d'hydrogène ou radical alcoyle de 1 à 6 atomes de carbone; et

$R^{10}$ représente un radical alcoyle de 1 à 6 atomes de carbone ou arylalcoyle de 7 à 10 atomes de carbone ou $R^9$ et $R^{10}$ pris ensemble avec l'atome d'azote auquel ils sont unis forment un hétérocycle pyrrolidinyle, imidazolidinyle, pipéridyle, pipérazinyle, 4-alcoylpipérazinyle dont le radical alcoyle compte 1 à 6 atomes de carbone ou morpholinyle, et

n représente l'un des entiers 0, 1 ou 2;

$R^1$ représente un radical pentafluorophényle, tétrafluorophényle ou

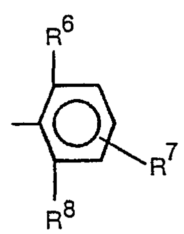

(III)

où $R^6$ et $R^8$ représentent indépendamment des atomes d'hydrogène ou radicaux alcoyle de 1 à 6 atomes de carbone, halo, trifluorométhyle, alcoxy de 1 à 6 atomes de carbone, alcoylthio de 1 à 6 atomes de carbone, cyano ou nitro;

$R^7$ représente un atome d'hydrogène ou radical alcoyle de 1 à 6 atomes de carbone, halo, trifluorométhyle, cyano ou nitro; ou

$R^8$ est tel que défini ci-dessus tandis que

$R^6$ et $R^7$ occupent la position ortho l'un par rapport à l'autre et, pris ensemble, représentent un radical butadiénylène, tétraméthylène ou triméthylène; ou un sel pharmaceutiquement acceptable de celui-ci.

2. Composé suivant la revendication 1, dans lequel $R^1$ représente un radical pentafluorophényle ou un radical de formule

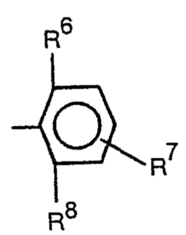

(III)

où $R^6$ et $R^7$ sont tels que définis dans la revendication 1 et $R^8$ représente un atome d'hydrogène.

3. Composé suivant la revendication 1 ou 2, dans lequel $R^3$ représente un radical alcoyle de 1 à 6 atomes de carbone ou

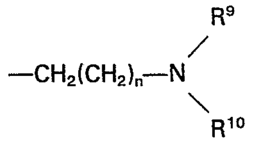

(II)

où n représente 1 ou 2, $R^{10}$ représente un radical benzyle ou phénéthyle et $R^9$ est tel que défini dans la revendication 1.

4. Composé choisi parmi l'ester méthylique d'acide 1,4,5,6,7,8-hexahydro-2,7-diméthyl-4-[2,3,4,5,6-pentafluorophényl]-5-oxo-1,7-naphtyridine-3-carboxylique, l'ester méthylique d'acide 1,4,5,6,7,8-hexahydro-2-méthyl-5-oxo-4-(2,3,4,5,6-pentafluorophényl)-7-(phénylméthyl)-1,7-naphthyridine-3-carboxylique et leurs sels pharmaceutiquement acceptables.

5. L'ester méthylique d'acide 1,4,5,6,7,8-hexahydro-2-méthyl-5-oxo-4-(2,3,4,5,6-pentafluorophényl)-1,7-naphtyridine-3-carboxylique ou un sel pharmaceutiquement acceptable de celui-ci.

6. Composé choisi parmi l'ester méthylique d'acide 1,4,5,6,7,8-hexahydro-2-méthyl-5-oxo-4-[2-(trifluorométhyl)phényl]-1,7-naphthyridine-3-carboxylique, son produit de substitution 7-méthylé ou 7-benzylé, l'ester 2-(N-benzyl-N-méthylamino)éthylique d'acide 1,4,5,6,7,8-hexahydro-2-méthyl-5-oxo-7-phénylméthyl-4-[2-(trifluorométhyl)phényl]-1,7-naphthyridine-3-carboxylique et leurs sels pharmaceutiquement acceptables.

7. Composé choisi parmi l'ester méthylique d'acide 1,4,5,6,7,8-hexahydro-2-méthyl-4-(2-méthylphényl)-5-oxo-1,7-naphthyridine-3-carboxylique, son produit de substitution 7-méthylé ou 7-benzylé et leurs sels pharmaceutiquement acceptables.

8. Ester méthylique d'acide 4-aryl-1,4,5,6,7,8-hexahydro-2-méthyl-5-oxo-1,7-naphthyridine-3-carboxylique (dont le radical aryle est un radical 3-nitrophényle, 3-(trifluorométhyl)phényle, 2-fluorophényle, 2-(n-butyl)phényle ou 2,3-diméthylphényle) ou ester méthylique d'acide 4-aryl-1,4,5,6,7,8-hexahydro-2,7-diméthyl-5-oxo-1,7-naphthyridine-3-carboxylique (dont le radical aryle est un radical 2-nitrophényle, 3-nitrophényle, 2-chlorophényle, 2,6-dichlorophényle, 2,5-diméthylphényle, 3-cyanophényle ou 2,3-diméthylphényle) ou ester méthylique d'acide 4-aryl-1,4,5,6,7,8-hexahydro-2-méthyl-5-oxo-7-(phénylméthyl)-1,7-naphtyridine-3-carboxylique (dont le radical aryle est un radical 2-(n-butyl)phényle, 2,3-diméthylphényle, 2-fluorophényle, 3-(trifluorométhyl)phényle ou 3-nitrophényle) ou un de leurs sels pharmaceutiquement acceptables.

9. Composé suivant l'une quelconque des revendications 1 à 8, à utiliser comme agent antihypertensif.

10. Composition pharmaceutique comprenant un composé suivant l'une quelconque des revendications 1 à 8 en association avec un excipient pharmaceutiquement acceptable.

11. Composition pharmaceutique suivant la revendication 10, sous la forme d'un comprimé ou d'une capsule.

12. Procédé de préparation d'un composé de formule:

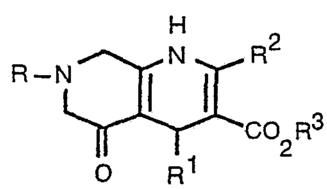

(I)

(où R, $R^1$, $R^2$ et $R^3$ sont tels que définis dans la revendication 1), ou d'un sel pharmaceutiquement acceptable de ce composé qui comprend

(a) la réaction d'un composé de formule IV

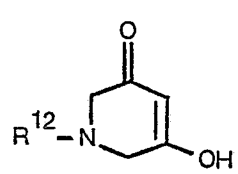

(IV)

(où $R^{12}$ représente un radical alcoyle de 1 à 6 atomes de carbone ou benzyle), d'un aldéhyde de formule $R^1CHO$ (où $R^1$ est tel que défini ci-dessus) et d'un ester 3-aminocrotonique substitué ou non substitué de formule VI

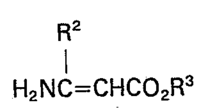

(VI)

(où $R^2$ et $R^3$ sont tels que définis ci-dessus); ou

(b) la réaction d'un composé de formule VIII

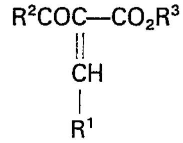

(VIII)

(où $R^1$, $R^2$ et $R^3$ sont tels que définis ci-dessus) avec un composé de formule IV

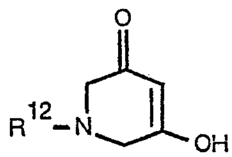

(IV)

(où $R^{12}$ est tel que défini ci-dessus) en présence d'ammoniac ou

(c) l'exécution sur un composé de formule VII

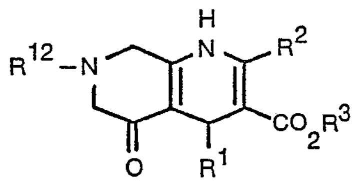

(VII)

(où $R^1$, $R^2$ et $R^3$ sont tels que définis ci-dessus et $R^{12}$ représente un radical benzyle) ou un sel de celui-ci d'une hydrogénolyse, et si la chose est souhaitée, la formation d'une forme base libre du composé de formule I par neutralisation d'un sel d'addition de celui-ci au moyen d'une base ou la formation d'un sel pharmaceutiquement acceptable par neutralization de la base libre au moyen d'un acide ou par exécution d'une double décomposition sur un autre sel.

13. Procédé suivant la revendication 12, dans lequel le stade (a) est exécuté et l'ester 3-aminocrotonique substitué ou non substitué est préparé *in situ* par ammonolyse de l'ester d'acide alcanoyl-acétique de formule $R^2COCH_2CO_2R^3$ (où $R^2$ et $R^3$ sont tels que définis dans la revendication 1).

**Revendications pour l'Etat Contractant AT**

1. Procédé de préparation d'un composé de formule:

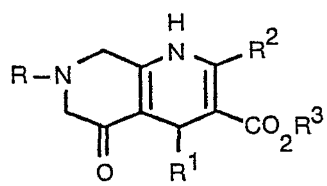

(I)

où R représente un atome d'hydrogène ou radical alcoyle de 1 à 6 atomes de carbone ou benzyle;

$R^2$ représente un radical alcoyle de 1 à 6 atomes de carbone;

$R^3$ représente un radical alcoyle de 1 à 6 atomes de carbone, alcoxyalcoyle dont chaque partie alcoyle compte 1 à 6 atomes de carbone, $—CH_2CF_3$, $—CH_2CH_2CF_3$ ou

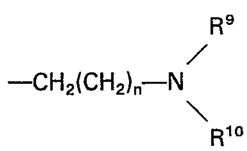

(II)

où $R^9$ représente un atome d'hydrogène ou radical alcoyle de 1 à 6 atomes de carbone; et

$R^{10}$ représente un radical alcoyle de 1 à 6 atomes de carbone ou arylalcoyle de 7 à 10 atomes de carbone ou $R^9$ et $R^{10}$ pris ensemble avec l'atome d'azote auquel ils sont unis forment un hétérocycle pyrrolidinyle, imidazolidinyle, pipéridyle, pipérazinyle, 4-alcoylpipérazinyle dont le radical alcoyle compte 1 à 6 atomes de carbone ou morpholinyle, et

n représente l'un des entiers 0, 1 ou 2;

$R^1$ représente un radical pentafluorophényle, tétrafluorophényle ou

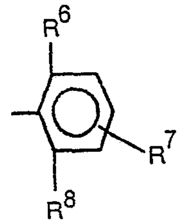

(III)

où $R^6$ et $R^8$ représentent indépendamment des atomes d'hydrogène ou radicaux alcoyle de 1 à 6 atomes de carbone, halo, trifluorométhyle, alcoxy de 1 à 6 atomes de carbone, alcoylthio de 1 à 6 atomes de carbone, cyano ou nitro;

$R^7$ représente un atome d'hydrogène ou radical alcoyle de 1 à 6 atomes de carbone, halo, trifluorométhyle, cyano ou nitro; ou

$R^8$ est tel que défini ci-dessus tandis que

$R^6$ et $R^7$ occupent la position ortho l'un par rapport à l'autre et, pris ensemble, un radical butadiénylène, tétraméthylène ou triméthylène, ou d'un sel pharmaceutiquement acceptable de celui-ci caractérisé par

(a) la réaction d'un composé de formule IV

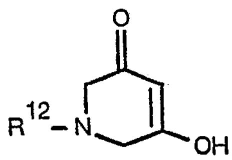

(IV)

(où $R^{12}$ représente un radical alcoyle de 1 à 6 atomes de carbone ou benzyle), d'un aldéhyde de formule $R^1CHO$ (où $R^1$ est tel que défini ci-dessus) et d'un ester 3-aminocrotonique substitué ou non substitué de formule VI

$R^2$
|
$H_2NC{=}CHCO_2R^3$ (VI)

(où $R^2$ et $R^3$ sont tels que définis ci-dessus); ou

(b) la réaction d'un composé de formule VIII

$R^2COC{-}CO_2R^3$
||
CH
|
$R^1$ (VIII)

(où $R^1$, $R^2$ et $R^3$ sont tels que définis ci-dessus) avec un composé de formule IV

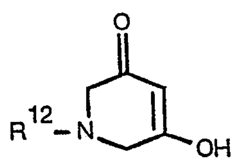

(IV)

(où $R^{12}$ est tel que défini ci-dessus) en présence d'ammoniac ou

(c) l'exécution sur un composé de formule VII

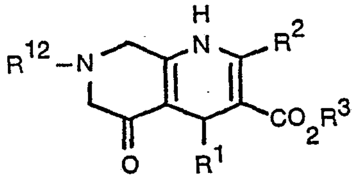

(VII)

(où $R^1$, $R^2$ et $R^3$ sont tels que définis ci-dessus et $R^{12}$ représente un radical benzyle) ou un sel de celui-ci d'une hydrogénolyse, et si la chose est souhaités, la formation d'une forme base libre du composé de formule I par neutralisation d'un sel d'addition de celui-ci au moyen d'une base ou la formation d'un sel

pharmaceutiquement acceptable par neutralisation de la base libre au moyen d'un acide ou par exécution d'une double décomposition sur un autre sel.

2. Procédé suivant la revendication 1, dans lequel le stade (a) est exécuté et l'ester 3-aminocrotonique substitué ou non substitué est préparé *in situ* par ammonolyse de l'ester d'acide alcanoylacétique de formule $R^2COCH_2CO_2R^3$ (où $R^2$ et $R^3$ sont tels que définis dans la revendication 1).

3. Procédé de préparation d'un composé de formule I

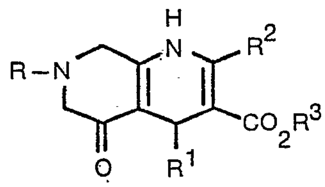

(I)

(où R, $R^1$, $R^2$ et $R^3$ sont tels que définis dans la revendication 1), caractérisé en ce qu'un sel d'addition d'acide de celui-ci est neutralisé au moyen d'une base.

4. Procédé de préparation d'un sel pharmaceutiquement acceptable de formule I

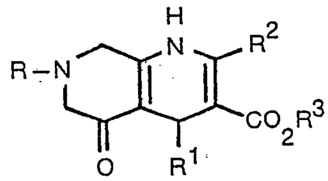

(I)

(où R, $R^1$, $R^2$ et $R^3$ sont tels que définis dans la revendication 1), caractérisé en ce que

(a) un composé de formule I tel que défini ci-dessus est neutralisé au moyen d'un acide, ou

(b) un autre sel du composé de formule I est soumis à la double décomposition.

5. Procédé suivant la revendication 4, caractérisé en ce que $R^1$ représente un radical tétrafluorophényle.

6. Procédé suivant la revendication 4, caractérisé en ce que $R^1$ représente un radical

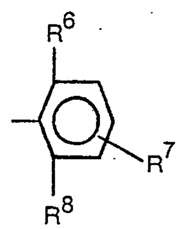

(III)

où $R^6$, $R^7$ et $R^8$ sont tels que définis dans la revendication 1.

7. Procédé de préparation d'un composé de formule I

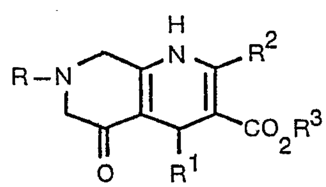

(I)

(où $R^1$ représente un radical pentafluorophényle et R, $R^2$ et $R^3$ sont tels que définis dans la revendication 1) ou d'un sel pharmaceutiquement acceptable de celui-ci, caractérisé par

(a) l'exécution d'une hydrogénolyse sur un composé de formule XII

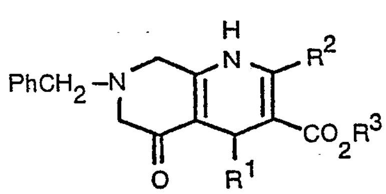

(XII)

(où $PhCH_2$ représente un radical benzyle et $R^1$, $R^2$ et $R^3$ sont tels que définis ci-dessus) ou un sel de celui-ci ou

(b) la réaction d'un composé de formule IV

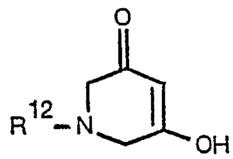

(IV)

(où $R^{12}$ représente un radical alcoyle de 1 à 6 atomes de carbone ou benzyle), d'un aldéhyde de formule $R^1CHO$ (où $R^1$ est tel que défini ci-dessus) et d'un ester 3-aminocrotonique substitué ou non substitué de formule VI

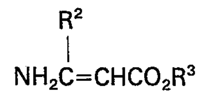

(VI)

(où $R^2$ et $R^3$ sont tels que définis ci-dessus) et si la chose est souhaitée, la conversion d'un composé de formule I en un sel pharmaceutiquement acceptable de celui-ci par neutralisation au moyen d'un acide.

8. Procédé de préparation d'un composé de formule I

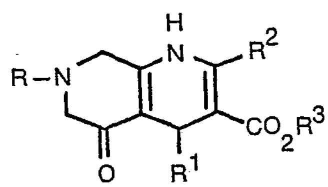

(I)

(où R, $R^2$ et $R^3$ sont tels que définis dans la revendication 1 et $R^1$ représente un radical de formule

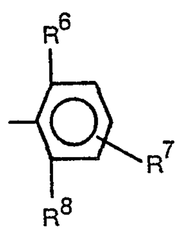

où $R^6$, $R^7$ et $R^8$ sont tels que définis dans la revendication 1) ou d'un sel pharmaceutiquement acceptable de celui-ci, caractérisé par

(a) l'exécution d'une hydrogénolyse sur un composé de formule XII

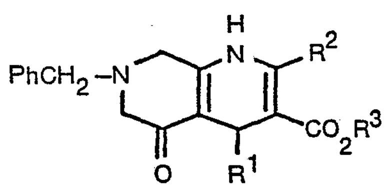

(XII)

(où $PhCH_2$ représente un radical benzyle et $R^1$, $R^2$ et $R^3$ sont tels que définis ci-dessus) ou un sel de celui-ci, ou

(b) la réaction d'un composé de formule IV

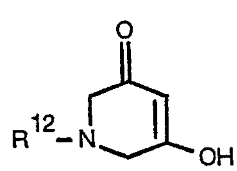

(IV)

(où $R^{12}$ représente un radical alcoyle de 1 à 6 atomes de carbone ou benzyle), d'un aldéhyde de formule $R^1CHO$ (où $R^1$ est tel que défini ci-dessus) et d'un ester 3-aminocrotonique substitué ou non substitué de formule VI

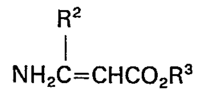

(VI)

(où $R^2$ et $R^3$ sont tels que définis ci-dessus) et, si la chose est souhaitée, la conversion d'un composé de formule I en un sel pharmaceutiquement acceptable de celui-ci par neutralisation au moyen d'un acide.

9. Préparation d'une composition pharmaceutique, caractérisée en ce qu'on associe un composé de formule I

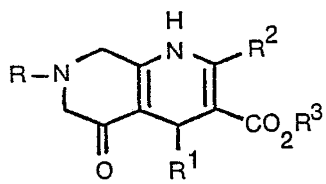

(I)

(où R, $R^1$, $R^2$ et $R^3$ sont tels que définis dans la revendication 1) avec un excipient pharmaceutiquement acceptable.

10. Procédé suivant la revendication 9, caractérisé en ce que la composition est préparée sous la forme d'un comprimé ou d'une capsule.

11. Procédé suivant la revendication 9 ou 10, dans lequel $R^1$ représente un radical de formule

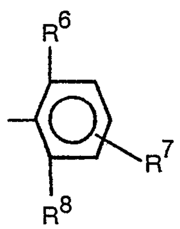

(III)

où $R^6$, $R^7$ et $R^8$ sont tels que définis dans la revendication 1.

12. Procédé suivant la revendication 9 ou 10, dans lequel $R^1$ représente un radical pentafluorophényle.